(19) Europäisches Patentamt
European Patent Office
Office européen des brevets

(11) Publication number: **0 082 668 A1**

# (12) EUROPEAN PATENT APPLICATION

(21) Application number: 82306686.5

(22) Date of filing: 14.12.82

(51) Int. Cl.$^3$: C 07 H 19/08
A 61 K 31/70

(30) Priority: 18.12.81 GB 8138323
03.03.82 GB 8206205
20.08.82 GB 8224034
25.09.82 GB 8227408

(43) Date of publication of application: 29.06.83 Bulletin 83/26

(84) Designated Contracting States: BE CH DE FR GB IT LI NL SE

(71) Applicant: BEECHAM GROUP PLC
Beecham House Great West Road
Brentford Middlesex(GB)

(72) Inventor: Harnden, Michael Raymond
47 Guildford Road
Horsham Sussex(GB)

(72) Inventor: Parkin, Anne
2 Fleur Gates Prince's Way
London(GB)

(72) Inventor: Luk, Kong
4 Palmer Close
Horley Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

## (54) 5-(2-Halogenovinyl)-2'-deoxyuridine derivatives, processes for their preparation, pharmaceutical compositions containing them and their use in treating viral infections.

(57) Compounds of formula (I):

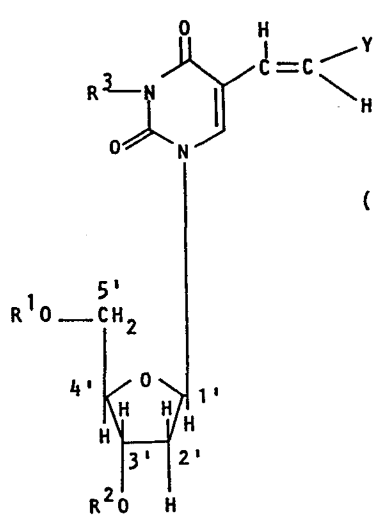

(I)

in which Y is a hydrogen, chlorine, bromine or iodine atom, preferably a bromine atom, each of $R^1$, $R^2$ and $R^3$ is a hydrogen atom or an acyl radical of the formula

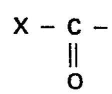

in which X is optionally unsaturated straight or branched chain $C_{1-18}$ alkyl, mono- or polycyclo $C_{1-18}$ alkyl, $C_{1-18}$ cycloalkylalkyl, phenyl optionally substituted with $C_{1-6}$ alkyl, $C_{1-6}$ alkoxy, nitro, optionally $C_{1-6}$ alkyl substituted amino, halogen or N,N-dialkylsulphonamido, or is straight or branched chain phenyl alkyl optionally substituted in the phenyl ring with $C_{1-6}$ alkyl, $C_{1-6}$ alkoxy, or halogen, with the provisos that at least one of $R^1$, $R^2$ and $R^3$ is an acyl radical, and that when $R^3$ is hydrogen, $R^1$ or $R^2$ is not

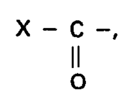

wherein X is $C_{1-6}$ alkyl, are useful in the treatment of infections caused by herpes viruses, such as herpes simplex types 1 and 2, and varicella.

EP 0 082 668 A1

# 5-(2-Halogenovinyl)-2'-deoxyuridine derivatives, processes for their preparation, pharmaceutical compositions containing them and their use in treating viral infections

This invention relates to certain deoxyuridine compounds which have antiviral activity.

U.K. Patent Specification No. 1,601,020 discloses E-5-(2-halogenovinyl)-2'-deoxyuridines which have antiviral activity selective against herpes virus.

We have now found a group of mono-, di- and trisubstituted E-5-(2-halogenovinyl)-2'-deoxyuridines which have antiviral activity, and which are useful in the treatment of infections caused by herpes viruses, such as herpes simplex type 1, herpes simplex type 2 and varicella.

According to the present invention there is provided a compound of formula (I):

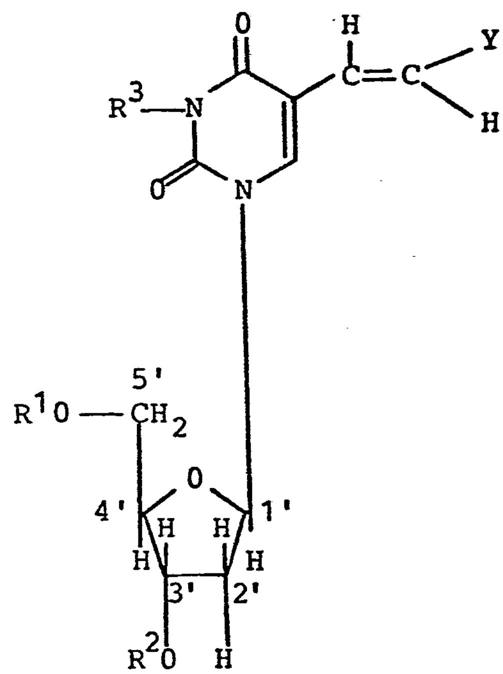

(I)

in which Y is a hydrogen, chlorine, bromine or iodine atom, preferably a bromine atom, each of $R^1$, $R^2$ and $R^3$ is a hydrogen atom or an acyl radical of the formula

$$X - \underset{\underset{O}{\|}}{C} -$$

in which X is optionally unsaturated straight or branched chain $C_{1-18}$ alkyl, mono- or polycyclo $C_{1-18}$

alkyl, $C_{1-18}$ cycloalkylalkyl, phenyl optionally substituted with $C_{1-6}$ alkyl, $C_{1-6}$ alkoxy, nitro, optionally $C_{1-6}$ alkyl substituted amino, halogen, or N,N-dialkylsulphonamido, or is straight or branched chain phenyl alkyl optionally substituted in the phenyl ring with $C_{1-6}$ alkyl, $C_{1-6}$ alkoxy, or halogen, with the provisos that at least one of $R^1$, $R^2$ and $R^3$ is an acyl radical, and that when $R^3$ is hydrogen, $R^1$ or $R^2$ is not $X-\underset{\underset{O}{\|}}{C}-$, wherein X is $C_{1-6}$ alkyl.

Preferably, X is a phenyl radical and is suitably optionally substituted in the para-position. When X is a saturated alkyl group, it is preferably a straight or branched $C_{1-6}$ alkyl group. When X is an unsaturated alkyl it is preferably a straight chain $C_{7-18}$ alkyl group containing one carbon to carbon double bond. When X is cycloalkyl or cycloalkylalkyl, it preferably has a total of up to twelve carbon atoms in the group.

Suitably, when $R^1$, $R^2$ and $R^3$ are simultaneously acyl radicals they are identical.

Compounds of formula (I) may be prepared by treating a compound of formula (II):

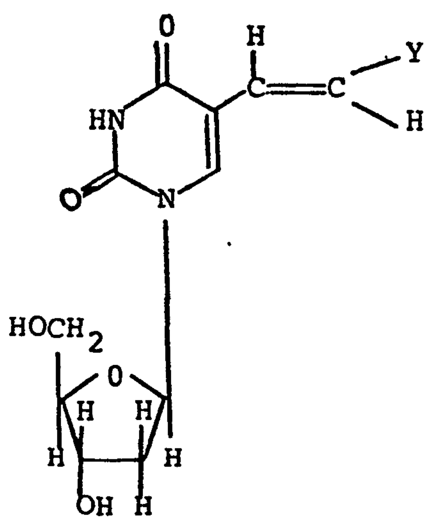

(II)

wherein Y is as defined in formula (I), with an acylating agent of formula $X-\underset{\underset{O}{\parallel}}{C}-L$ wherein L is a good leaving group, such as a halogen atom or a carboxylate group. Suitably these acylating agents are acid halides, or symmetrical or unsymmetrical anhydrides. Preferably when the acylating agent is an acid halide, L is a chlorine atom.

The acylation reaction will generally produce a mixture of reaction products. The extent of acylation will depend on a number of factors, such as the relative amounts and chemical natures of reactants, the physical conditions of the reaction, and the solvent system. The mixture may separate into its pure components on using standard chromatographic techniques.

3,3',5'-Triacylated compounds of formula (I), ie those in which $R^1$, $R^2$ and $R^3$ are all acyl groups of formula $X-\underset{\underset{O}{\parallel}}{C}-$ wherein X is as defined in formula (I), may be prepared by treating a compound of formula (II) with a large excess of an acylating agent of formula $X-\underset{\underset{O}{\parallel}}{C}-L$.

Suitably the molar ratio of reactants is about 4:1 of acylating agent to substrate.

The reaction is suitably carried out in an anhydrous polar organic solvent, preferably anhydrous pyridine, at room temperature.

The product is preferably purified by recrystallisation from ethanol.

5'-Monoacylated compounds of formula (I), in which $R^1$ is an acyl group and $R^2$, $R^3$ are hydrogen atoms, and 3',5'-diacylated compounds of formula (I) in which $R^1$ and $R^2$ are acyl groups may be prepared in a similar manner as the triacylated compound described above but using lower molar ratios of the compound of formula $X-\underset{O}{\underset{\|}{C}}-L$ to starting compound of formula (II). The molar ratio of reactants is suitably about 1:1 for the preparation of 5'-mono- acylated compounds of formula (I). Suitably 2 to 3 molar equivalents of the acylating agent may be used in the preparation of the 3',5'-diacylated compounds of formula (I). The reaction is suitably carried out in anhydrous pyridine at room temperature, and the product purified by re-crystallisation from ethanol, or by standard chromatographic techniques.

3'-Monoacylated compounds of formula (I) in which $R^2$ is an acyl group and both $R^1$ and $R^3$ are hydrogen atoms, may be prepared by treating a compound of formula (III):

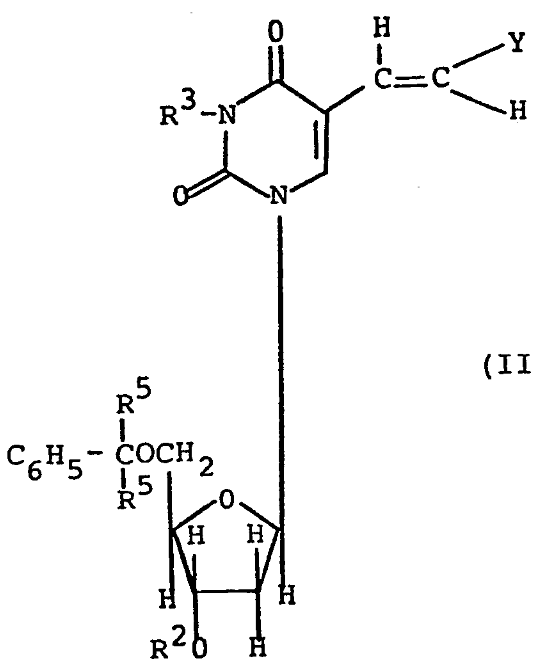

(III)

wherein $R^2$ is an acyl group, $R^3$ is hydrogen, $R^5$ is phenyl or para-methoxy phenyl, and Y is as defined in formula (I), with an acid, preferably acetic acid, at room temperature or slightly above room temperature.

The product may be purified chromatographically; suitably by preparative thin layer chromatography.

3,3'-Diacylated compounds of formula (I) in which both $R^2$ and $R^3$ are acyl groups, and $R^1$ is hydrogen may be prepared by treating a compound of formula (III) wherein $R^2$ and $R^3$ are both acyl groups as defined in formula (I) and $R^5$ is phenyl or para-methoxy phenyl, under the same conditions as described for the preparation of the 3'-monoacyl derivatives above.

The product is preferably purified chromatographically by, for example, column chromatograpy on silica gel.

The acylating agents of formula XCO.L are either known compounds or can be prepared from known compounds by conventional means.

The compounds of formula (III) wherein $R^2$ is an acyl group and $R^3$ is either a hydrogen atom or an acyl group, may be prepared by treating a 5'-O-trityl or a 5'-O-dimethoxytrityl compound of formula (IV):

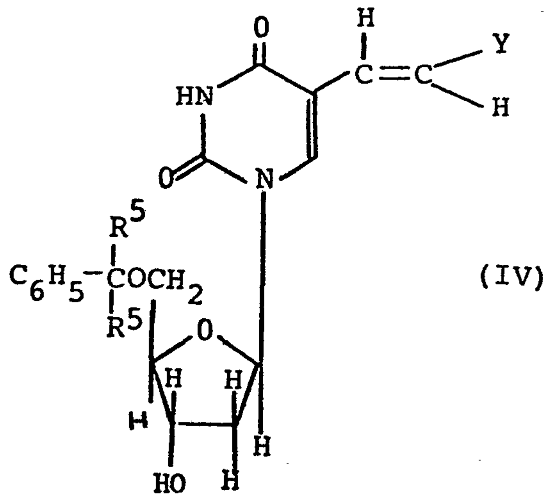

(IV)

with a compound of formula $X-\underset{\underset{O}{\|}}{C}-L$. The reaction conditions are similar to those described above for the preparation of the 3,3',5'-triacylated compounds, preferably using an approximately 3:1 molar ratio of $X-\underset{\underset{O}{\|}}{C}-L$ to compounds of formula (IV). The resultant mixture may be separated using standard chromatographic techniques in order to afford a pure sample of either the 3'-mono- or 3,3'-diacylated compounds of formula (III).

The compounds of formulae (III) and (IV) are important intermediates in the preparation of the 3,3'-di- and 3'-monoacylated compounds and are themselves novel compounds which form a further aspect of the present invention.

The compounds of formula (IV) may be prepared by treating a compound of formula (II) as defined above, with a triphenylhalomethane compound, preferably triphenyl chloromethane. The reaction is suitably carried out in an anhydrous polar organic solvent,

preferably at room temperature. The product may be purified by column chromatography on silica gel.

3-Monoacylated compounds of formula (I) in which $R^3$ is an acyl group and $R^1$, $R^2$ are both hydrogen, may be prepared by selective saponification of 3,3',5'-triacylated compounds of formula (I) in which $R^1$, $R^2$ and $R^3$ are all acyl groups. The reaction is suitably carried out in a polar organic solvent, preferably a lower alcohol, for example methanol, containing a suitable base, preferably an alkali metal lower alkoxide, for example sodium methoxide, at room temperature. The product is preferably purified chromatographically by, for example, column chromatography on silica gel.

3 ,5'-diacylated compounds of formula (I) may be prepared by treating a 3-monoacylated compound with approximately one equivalent of acylating agent, and separating the desired compound from the resultant mixture, preferably by chromatographic techniques.

An alternative process for the preparation of 3-monoacylated compounds of formula (I) comprises treating a compound of formula (V):

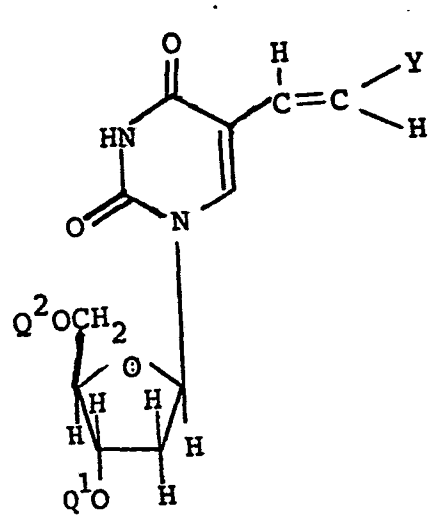

(V)

wherein $Q^1$ and $Q^2$ are O-protecting groups, with the acylating agent of formula $X-\underset{\underset{O}{\|}}{C}-L$, and deprotecting the resultant product.

Preferably, $Q^1$ and $Q^2$ are each trialkylsilyl groups, such as trimethylsilyl.

The compound of formula (V) may be prepared by treating a a compound of formula (II) as defined above, with a silylating agent.

A preferred silylating agent is N,O-bistrimethylsilylacetamide, and the silylation reaction is preferably carried out in an anhydrous polar organic solvent, suitably tetrahydrofuran, at room temperature.

The removal of the O-protecting silyl groups may be performed using conventional procedures, suitably by partitioning the silylated compound between chloroform and dilute hydrochloric acid. The product may be purified chromatographically by for example, column chromatography on silica gel.

The compounds of formula (I) may be formulated for use in a pharmaceutical composition. Accordingly, in a further aspect of the invention, there is provided a pharmaceutical composition which comprises a compound of the formula (I) together with a pharmaceutically acceptable carrier or excipient.

Compositions which may be administered by the oral route to humans may be compounded in the form of syrups, tablets and capsules. When the composition is in the form of a tablet, any pharmaceutical carrier suitable for formulating such solid compositions may be

used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring may be added. The composition may also be presented with a sterile liquid carrier for injection, suitably intramuscular injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop compositions well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound depends on the particular compound employed, but is in general in the range of form 1.0 to 20 mg/kg of body weight per day or more usually 2.0 to 10 mg/kg per day.

The present invention further provides a method of treating mammals, including humans, suffering from viral infections, which comprises administering to the sufferer a pharmaceutically effective, non-toxic, amount of a compound of formula (I).

The following Examples illustrate the invention.

Example 1

Preparation of 3-N-benzoyl-E-5-(2-bromovinyl)-3',5'-di-O-benzoyl-2'-deoxyuridine

To a solution of E-5-(2-bromovinyl)-2'-deoxyuridine (1g, 3 mmol) in dry pyridine (15 mL) at room temperature, benzoyl chloride (1.4 mL, 12.1 mmol) was added dropwise. The solution was stirred overnight (20h) and then poured into ice/water (200 mL) and the resulting precipitate filtered, washed with water and recrystallised from ethanol to give 3-N-benzoyl-E-5-(2-bromovinyl)-3',5'-di-O-benzoyl-2'-deoxyuridine (1.2g, 86%), mp 159-160°C.

$R_f$ 0.71[a] (ethyl acetate/cyclohexane, 1:1); $\nu_{max.}$(KBr), 1750, 1720, 1710, 1670 (C=O)$cm^{-1}$; $^1$Hnmr ($CDCl_3$) $\delta$ 2.50 (2H, m, 2'-$CH_2$), 4.53 (1H, m, 4'-CH), 4.74 (2H, m, 5'-$CH_2$), 5.60 (1H, m, 3'-CH), 6.18 (1H, d, J=13.5 Hz, CH=CHBr), 6.37 (1H, d.d, $J_1$=8 Hz, $J_2$=6 Hz, 1'-CH), 7.10-8.18 (17H, m, 3 x $C_6H_5$, 6-CH, CH=CHBr).

Analysis

Found: C, 59.60; H, 4.00; N, 4.24% $C_{32}H_{25}BrN_2O_8$
requires: C, 59.55; H, 3.90; N, 4.34%.

[a]Note: $R_f$ values were measured on Merck silica gel $60_f$ precoated plates, layer thickness 0.5 mm.

Example 2

Preparation of E-5-(2-bromovinyl)-3',5'-di-O-benzoyl-2'-deoxyuridine

To a solution of E-5-(2-bromovinyl)-2'-deoxyuridine (1g, 3.0 mmol) in dry pyridine (15 mL) benzoyl chloride (o.8 mL, 6.9 mmol) was added dropwise. The solution was stirred at room temperature overnight, poured into ice/water (200 mL) and the resulting precipitate filtered, washed with water and recrystallised from ethanol to give E-5-(2-bromovinyl)-3'-5'-di-O-benzoyl-2'-deoxyuridine (0.85 g, 52%); mp 177-178°C.

$R_f$ 0.63 (ethyl acetate/cyclohexane 1:1);
$\nu_{max}$ (KBr), 3260 (NH), 1730, 1700 (C=O)$cm^{-1}$;
$^1$Hnmr ($CDCl_3$) $\delta$ 2.52 (2H, m, 2'-$CH_2$), 4.58 (1H, m, 4'-CH), 4.63 (2H, m, 5'-$CH_2$), 5.60 (1H, m, 3'-CH), 6.15 (1H, d, J=13.5 Hz, CH-CHBr), 6.38 (1H, m, 1'-CH), 7.1-8.2 (12H, m, 2 x $C_6H_5$, 6-CH, CH=CHBr), 9.20 (1H, s, $D_2O$ exchangeable, NH).

Analysis

Found: C, 55.53; H, 3.67; N, 5.20% $C_{25}H_{21}BrN_2O_7$
requires: C, 55.47; H, 3.91; N, 5.17%

## Example 3

### Preparation of 5'-O-benzoyl-E-5-(2-bromovinyl)-2'-deoxyuridine

To a solution of E-5-(2-bromovinyl)-2'-deoxyuridine (1.04 g, 3.1 mmol) in dry pyridine (15 mL), benzoyl chloride (0.4 mL, 3.4 mmol) was added dropwise. The solution was stirred at room temperature for 1.5 hours, then poured into ice/water (200 mL) and the resulting precipitate filtered, washed with water and recrystallised from ethanol to give 5'-O-benzoyl-E-(2-bromovinyl)-2'-deoxyuridine (0.44 g, 32%), mp 196-197°C.

$R_f$ 0.08 (ethyl acetate/cyclohexane 1:1); $\nu_{max.}$ (KBr) 3500-3000 (NH,OH), 1720, 1685 (C=O)$cm^{-1}$; $^1$Hnmr [$(CD_3)_2SO$] $\delta$ 2.23 (2H, m, 2'-$CH_2$), 4.08 (1H, m, 4'-CH), 4.40 (3H, m, 3'-CH, 5'-$CH_2$), 5.43 (1H, d, J=4Hz, OH exchangeable with $D_2O$), 6.15 (1H, m, 1'-CH), 6.65 (1H, d, J=13.5 Hz, CH=CHBr), 7.23 (1H, d, J=13.5 Hz, CH=CHBr), 7.45 (6H, m, $C_6H_5$, 6-CH), 11.53 (1H, s, $D_2O$ exchangeable, NH).

### Analysis

Found: C, 49.33; H, 3.89; N, 6.30% $C_{18}H_{17}BrN_2O_6$ requires: C, 49.44; H, 3.92; N, 6.41%

Example 4

Preparation of E-5-(2-bromovinyl)-5'-O-trityl-2'-deoxyuridine (Intermediate compound of formula (IV) wherein Y is Br)

A solution of E-5-(2-bromovinyl)-2'-deoxyuridine (0.5 g, 1.5 mmol) and triphenylchloromethane (0.6 g, 1.53 mmol) in anhydrous pyridine (5 mL) was stirred at room temperature for 20 hours. The reaction mixture was poured into dichloromethane/water (50:50 mL) and the aqueous layer was separated and further extracted with dichloromethane (2 x 50 mL). The combined dichloromethane extracts were washed with cold, saturated sodium hydrogen carbonate solution (50 mL), saturated sodium chloride solution (50 mL), and then combined and dried ($Na_2SO_4$). Evaporation of the solvent in vacuo, followed by codistillisation of the residue with carbon tetrachloride (50 mL), afforded a yellow oil (1.1 g) which was purified by column chromatography on silica gel (30 g). Elution with ethyl acetate/hexane (85:15) (100 mL) gave the 5'-O-trityluridine (0.69 g, 80%). A sample recrystallised from dichloromethane/hexane had mp 165-67°C.
Tlc: $R_f$ = 0.5 in ethyl acetate;
$\nu_{max.}$ (KBr) 3430 (NH,OH), 1695 (C=O) $cm^{-1}$;
$\lambda_{max.}$ (EtOH) 294 nm ($\varepsilon$, 10,500); 250 nm ($\varepsilon$ 13,100);
$^1$Hnmr (DMSO $d_6$) $\delta$ 2.2 (2H, m, 2'-$CH_2$), 3.2 (2H, m, 5'-$CH_2$), 3.84 (1H, m, 4'-CH), 4.25 (1H, m, 3'-CH), 5.28 (1H, m, 3'-CH), 6.12 (1H, t, J=7 Hz, 1'-CH), 6.44 (1H, d, J=14 Hz, CH=CHBr), 7.4 (16H, m, $(C_6H_5)_3C$, CH=CHBr), 7.72 (1H, s, 6-CH), 11.62 (1H, m, NH);
m/e 243 (22%), 183 (69), 165 (45), 154 (27), 105 (100).

Analysis

Found: C, 62.22; H, 4.86; N, 4.57% $C_{30}H_{27}N_2O_5Br$ requires: C, 62.65; H, 4.73; N, 4.87%.

Example 5

E-5-(2-Bromovinyl)-5'-O-dimethoxytrityl-2'-deoxyuridine

To a solution of E-5-(2-bromovinyl)-2'-deoxyuridine (10 g, 30 mmol) in anhydrous N,N-dimethylformamide (100 mL), containing triethylamine (7 mL, 45 mmol) and 4-dimethylaminopyridine (0.30 g, 2.5 mmol) was added 4,4'-dimethoxytrityl chloride (12 g, 35 mmol) and the resultant solution stirred at 25°C overnight. The solution was poured onto ice/water (300 mL) and the aqueous mixture extracted with methylene chloride (2 x 200 mL). The combined organic extracts were washed with saturated ammonium chloride solution (200 mL), water (200 mL) and dried ($MgSO_4$). Removal of the solvent under reduced pressure gave the crude product which was purified by column chromatography to give E-5-(2-bromovinyl)-5'-O-dimethoxytrityl-2'-deoxyuridine (14.75 g, 77%) as a solid with mp 139°C.

$\nu_{max.}$(KBr) 1703, 1506, 1250, 830, 755 $cm^{-1}$;
$^1$Hnmr ($CDCl_3$) $\delta$ 2.45 (2H, m, 2'-$CH_2$), 3.35 (1H, br.s, $D_2O$ exchangeable, 3'-OH), 3.50 (2H, m, 5'-$CH_2$), 3.80 (6H, s, 2 x $CH_3O$), 4.20 (1H, m, 4'-CH), 4.65 (1H, m, 3'-CH), 5.95 (1H, d, CH=CHBr, J=14 Hz), 6.40 (1H, m, 1'-CH), 6.70-7.60 (14H, m, $(CH_3OC_6H_4)_2C$, $(C_6H_5)C$, CH=CHBr), 7.80 (1H, s, 6-CH), 9.85 (1H, br.s, $D_2O$ exchangeable, NH).

Examples 6 and 8

3-N-Benzoyl-3'-O-benzoyl-E-5-(2-bromovinyl)-5'-O-trityl-2'-deoxyuridine and 3'-O-Benzoyl-E-5-(2-bromovinyl)-5'-O-trityl-2'-deoxyuridine

To a solution of E-5-(2-bromovinyl)-5'-O-trityl-2'-deoxyuridine (1.52 g, 2.6 mmol) in dry pyridine (40 mL) was added benzoyl chloride (0.93 mL, 8.0 mmol). The solution was poured into ice/water (200 mL) and the resulting sticky solid extracted with ethyl acetate (2 x 100 mL). The organic phase was washed with hydrochloric acid (5M) (50 mL), followed by saturated sodium bicarbonate solution and finally water. It was then dried (magnesium sulphate) and the solvent removed to give an oil which could not be crystallised and was column chromatographed on silica, eluting with ethyl acetate/cyclohexane 1:3. The following two compounds were isolated by this method.

| Example 6 | Yield | mp/°C |
|---|---|---|
| 3-N-Benzoyl-3'-O-benzoyl-E-5-(2-bromovinyl)-5'-O-trityl-2'-deoxyuridine (recrystallised from ethanol) | 36% | 111-112 |

Tlc $R_f$ = 0.34 in ethyl acetate/cyclohexane 1:3;
$\nu_{max}$ (KBr) 1750, 1710, 1670 (C=O) $cm^{-1}$;
$^1$Hnmr ($CDCl_3$) $\delta$ 2.66 (2H, m, 2'-$CH_2$), 3.60 (2H, m, 5'-$CH_2$), 4.33 (1H, m, 4'-CH), 5.80 (2H, m, 3'-CH, CH=CHBr), 6.51 (1H, m, 1'-CH), 7.0-8.2 (27H, m, 5 x $C_6H_5$, 6-CH, CH=CHBr).

Analysis

Found: C, 67.14; H, 4.47; N, 3.60% $C_{45}H_{55}BrN_2O_7$ requires: C, 66.93; H, 4.57; N, 3.63%

Example 8

| 3'-O-Benzoyl-E-5-(2-bromovinyl)- | Yield | mp/°C |
|---|---|---|
| 5'-O-trityl-2'-deoxyuridine | 23% | 239-240 |
| (recrystallised from carbon tetra-chloride/ethyl acetate | | |

Tlc $R_f$ = 0.17 in ethyl acetate/cyclohexane 1:3; $\vartheta_{max}$ (KBr) 3100-2800 (NH), 1720, 1680 (C=O) $cm^{-1}$; $^1$Hnmr ($CDCl_3$) $\delta$ 2.62 (2H, m, 2'-$CH_2$), 3.55 (2H, m, 5'-$CH_2$), 4.35 (1H, m, 4'-CH), 3.80 (2H, m, 3'-CH, CH=CHBr), 6.45 (1H, m, 1'-CH), 7.0-8.2 (22H, 4 x $C_6H_5$, 6-CH, CH=CHBr), 8.75 (1H, s, $D_2O$ exchangeable, NH).

Analysis

Found: C, 64.56; H, 4.79; N, 3.98% $C_{36}H_{31}BrN_2O_6$
requires: C, 64.77; H, 4.68; N, 4.20%

Examples 7 and 9

Preparation of 3-N-benzoyl-3'-O-benzoyl-E-5-(2-bromovinyl)-5'-O-dimethoxytrityl-2'-deoxyuridine and 3'-O-benzoyl-E-5-(2-bromovinyl)-5'-O-dimethoxytrityl-2'-deoxyuridine

To a solution of E-5-(2-bromovinyl)-5'-O-dimethoxytrityl-2'-deoxyuridine (2.4 g, 3.8 mmol) in dry pyridine (40 mL), benzoyl chloride (1.1 mL, 9.5 mmol) was added dropwise. The solution was stirred at room temperature overnight and then poured into ice/water (200 mL) and the resulting precipitate filtered, washed with water and purified by column chromatography on silica eluting with ethyl acetate/cyclohexane 1:3. The following two compounds were isolated by this method:

| Example 7 | Yield | mp/°C |
|---|---|---|
| 3-N-Benzoyl-3'-O-benzoyl-E-5-(2-bromovinyl)-5'-O-dimethoxytrityl-2'-deoxyuridine (recrystallised from ethanol) (light sensitive) | 40% | 116-117 |

Tlc $R_f$ 0.4 in ethyl acetate/cyclohexane 1:3;
$\nu_{max.}$ (KBr) 1760, 1725, 1710, 1670 (C=O) $cm^{-1}$;
$^1$Hnmr ($CDCl_3$) $\delta$ 2.68 (2H, m, 2'-$CH_2$), 3.57 (2H, m, 5'-$CH_2$), 3.80 (6H, s, 2 x $CH_3O$), 4.34 (1H, m, 4'-CH), 5.75 (2H, m, 3'-CH, CH=CHBr), 6.48 (1H, m, 1'-CH), 6.7-8.1 (25H, 3 x $C_6H_5$, 2 x $C_6H_4$, 6-CH, CH=CHBr).

Analysis

Found: C, 65.59; H, 4.64; N, 3.22% $C_{46}H_{39}BrN_2O_9$.
requires: C, 65.48; H, 4.66; N, 3.32%

Example 9

3'-O-Benzoyl-E-5-(2-bromovinyl)-
5'-O-dimethoxytrityl-2'-deoxyuridine 13% 164-165
(recrystallised from ethanol)
(light sensitive)

Tlc $R_f$ 0.24 in ethyl acetate/cyclohexane 1:3; $\nu_{max}$ (KBr) 3200 (NH), 1720, 1690 (C=O) $cm^{-1}$; $^1$Hnmr ($CDCl_3$) $\delta$ 2.60 (2H, m, 2'-$CH_2$), 3.50 (2H, m, 5'-$CH_2$), 3.78 (6H, s, 2 x $CH_3O$), 4.30 (1H, m, 4'-CH), 5.78 (2H, m, 3'-CH, CH=CHBr), 6.46 (1H, m, 1'-CH), 6.7-8.2 (20H, m, 2 x $C_6H_5$, 2 x $C_6H_4$, 6-CH, CH=CHBr), 9.05 (1H, s, $D_2O$ exchangeable, NH).

Analysis

Found: C, 63.26; H, 4.77; N, 3.68% $C_{39}H_{35}BrN_2O_8$.
requires: C, 63.33; H, 4.77; N, 3.79%

Examples 10 and 11

General method for the acetic acid deprotection of 3-N-Benzoyl-3'-O-benzoyl-E-5-(2-bromovinyl)-5'-dimethoxytrityl-2'-deoxyuridine and 3'-O-benzoyl-E-5-(2-bromovinyl)-5'-O-dimethoxytrityl-2'-deoxyuridine

A suspension of E-5-(2-bromovinyl)-5'-dimethoxytrityl-3-N-benzoyl-3'-O-benzoyl-2'-deoxyuridine or the 3'-O-benzoyl ester (1.2-1.5 mmol) in 80% acetic acid (40-50 mL) was stirred at room temperature for 45-90 minutes. The solvent was removed and the residue column chromatographed on silica, eluting with ethyl acetate/ cyclohexane 1:3 in the case of Example 10 and ethyl acetate/cyclohexane 1:1, increasing polarity to ethyl acetate/ethanol 20:1 in the case of Example 11. The following compounds were prepared by this method:

| Example 10 | Yield | mp/°C |
|---|---|---|
| 3-N-Benzoyl-3'-O-benzoyl-E-5-(2-bromovinyl)-2'-deoxyuridine (recrystallised from ethanol/ water) | 78% | 102-103 |

Tlc $R_f$ 0.42 in ethyl acetate/cyclohexane 1:1;
$\nu_{max}$ (KBr) 3060, 2940 (OH), 1750, 1705, 1670 (C=O)$cm^{-1}$
$^1$Hnmr ($CDCl_3$) $\delta$ 2.52 (3H, m, 2'-$CH_2$,$D_2O$ exchangeable, 5'-OH), 4.00 (2H, m, 5'-$CH_2$), 4.28 (1H, m, 4'-CH), 6.43 (1H, m, 1'-CH), 6.73 (1H, d, J=13.5 Hz, CH=CHBr), 7.3-8.3 (12H, m, 2 x $C_6H_5$, CH=CHBr, 6-CH).

Analysis

Found: C, 55.11; H, 3.92; N, 4.78% $C_{25}H_{21}BrN_2O_7$.
requires: C, 55.47; H, 3.91; N, 5.17%

Example 11

3'-O-Benzoyl-E-5-(2-bromovinyl)-
2'-deoxyuridine 65% 222-223
(recrystallised from methanol)

Tlc $R_f$ 0.49 in ethyl acetate/cyclohexane 2:1; $\nu_{max.}$ (KBr) 3500-2800 (NH, OH), 1710, 1680 (C=O)$cm^{-1}$; $^1$Hnmr [$(CD_3)_2SO$] $\delta$ 2.50 (m, 2'-$CH_2$), 3.77 (2H, m, 5'-$CH_2$), 4.24 (1H, m, 4'-CH), 5.30 (1H, br.s, $D_2O$ exchangeable, OH), 5.54 (1H, m, 3'-CH), 6.32 (1H, d, J=13.5 Hz, CH=CHBr), 6.34 (1H, d, J=13.5 Hz, CH=CHBr), 7.4-8.2 (6H, m, $C_6H_5$, 6-CH), 11.69 (1H, br.s, $D_2O$ exchangeable, NH).

Analysis

Found: C, 49.03; H, 3.85; N, 6.13% $C_{18}H_{17}BrN_2O_6$.
requires: C, 49.44; H, 3.92; N, 6.41%

Example 12

Preparation of 3-N-Benzoyl-E-5-(2-bromovinyl)-2'-deoxyuridine

A suspension of 3-N-benzoyl-E-5-(2-bromovinyl)-3', 5'-di-O-benzoyl-3'-deoxyuridine (0.65 g, 1.0 mmol) in 0.2M methanolic sodium methoxide solution (60 mL, 1.2 mmol) was stirred at room temperature for 45 minutes. After this time the solutin was almost clear and was neutralised with Amberlite IR 200 (H), filtered, the solvent removed and the residue column chromatographed on silica eluting with chloroform/ethanol (20:1) to give 3-N-benzoyl-E-5-(2-bromovinyl)-2'-deoxyuridine (0.31 g, 70%), recrystallised from ethanol/water, mp 171-172°C.

Tlc $R_f$ 0.1 (chloroform/ethanol 20:1);
$\nu_{max.}$(KBr), 3500-2900 (OH), 1755, 1705, 1660 (C=O)$cm^{-1}$;
$^1$Hnmr [$(CD_3)_2SO$] $\delta$ 2.25 (2H, m, 2'-$CH_2$), 3.66 (2H, m, 5'-$CH_2$), 3.80 (1H, m, 4'-CH), 4.33 (1H, m, 3'-CH), 5.15 (1H, t, J=5.5 Hz, $D_2O$ exchangeable, 5'-OH), 5.26 (1H, d, J=5 Hz, $D_2O$ exchangeable, 3'-OH), 6.10 (1H, t, J=6.5Hz, 1'-CH), 6.93 (1H, d, J=13.5 Hz, CH=CHBr), 7.24 (1H, d, J=13.5 Hz, CH=CHBr), 7.4-8.2 (5H, m, $C_6H_5$), 8.35 (1H, s, 6-CH).

Analysis

Found: C, 49.09; H, 3.90; N, 6.29% $C_{18}H_{17}BrN_2O_6$.
requires: C, 49.44; H, 3.92; N, 6.41%

Example 13

E-5-(2-bromovinyl)-3-N-p-chlorobenzoyl-3',5'-di-O-p-chlorobenzoyl-2'-deoxyuridine

The title product was prepared by the method described for Example 1, using E-5-(2-bromovinyl)-2'-deoxyuridine (1.4 g, 4 mmol) in dry pyridine (25 mL) and p-chlorobenzoyl chloride (2.1 mL, 16.6 mmol). The product was purified by column chromatography on silica eluting with ethyl acetate/cyclohexane (1:4) giving 2.2 g (67%), which was recrystallised from ethanol/water mp 107-8°C.

Rf = 0.63 in ethyl acetate/cyclohexane (1:1).

$\nu_{max}$ (KBr), 1750, 1720, 1670 (C=O)$cm^{-1}$;

$^1$Hnmr ($CDCl_3$) $\delta$ 2.56 (2H, m, 2'-$CH_2$), 4.67 (3H, m, 5'-$CH_2$, 4'-CH), 5.63 (1H, m, 3'-CH), 6.34 (2H, m, 1'-CH, CH=CHBr), 7.15-8.2 (14H, m, 3x$C_6H_4$, 6-CH, CH=CHBr).

Analysis

Found: C, 51.20; H, 2.86; N, 3.64% $C_{32}H_{22}BrCl_3N_2O_8$.
requires: C, 51.33; H, 2.96; N, 3.74%.

Example 14

E-5-(2-Bromovinyl)-3-N-p-methoxybenzoyl-3',5'-di-O-p-methoxybenzoyl-2'-deoxyuridine

The title product was prepared by the method described for Example 1 using E-5-(2-bromovinyl)-2'-deoxyuridine (0.58 g, 1.7 mmol) in dry pyridine (10 mL) dimethylaminopyridine (0.1 g) and p-methoxybenzoyl chloride (2.85 mL, 21 mmol). The product was purified by column chromatography on silica eluting with cyclohexane/ethyl acetate (4:1) giving 0.9 g (70%) which was recrystallised from ethanol/acetone, mp 104-5°C.

Rf = 0.32 in ethyl acetate/cyclohexane (1:1)

$\nu_{max.}$ (KBr) 1740, 1710, 1665 (C=O) $cm^{-1}$

$^1$Hnmr ($CDCl_3$) $\delta$ 2.53 (2H, m, 2'-$CH_2$), 3.90 (9H, s, 3x$CH_3O$), 4.73 (3H, m, 5'-$CH_2$, 4'-CH), 5.67 (1H, m, 3'-CH), 6.24 (1H, d, J=13.5 Hz, CH=CHBr), 6.43 (1H, m, 1'-CH), 6.88-8.20 (14H, m, 3x$C_6H_4$, 6-CH, CH=CHBr).

Analysis

Found: C, 57.00; H, 4.30; N, 3.77%. $C_{34}H_{31}BrN_2O_{11}$ requires: C, 57.15; H, 4.25; N, 3.81%.

Example 15

E-5-(2-Bromovinyl)-3',5'-di-O-p-chlorobenzoyl-2'-deoxyuridine

The title product was prepared by the method described for Example 2 using E-5-(2-bromovinyl)-2'-deoxyuridine (1.13 g, 3.4 mmol) in dry pyridine (15 mL) and p-chlorobenzoyl chloride (0.95 mL, 7.5 mmol). After stirring at room temperature for 18 hours, the solution was poured into water/ice (200 mL), and extracted with two 100 mL portions of ethyl acetate followed by 50 mL chloroform. The combined organic layers were washed with two 50 mL portions of 5N hydrochloric acid, two 50 mL portions of saturated sodium carbonate and 50 mL distilled water. After drying over magnesium sulphate, the solvent was removed and the residue columned on silica eluting with ethyl acetate/cyclohexane (1:1) to give 0.95 g (46%) which was recrystallised from acetone/water, mp 230-1°C(dec).
Rf = 0.44 in ethyl acetate/cyclohexame (1:1)
$\nu_{max}$ (KBr) 3300-3000 (NH), 1725, 1705, 1680 (C=O) $cm^{-1}$
$^1$Hnmr [$(CD_3)_2SO$]$\delta$ 2.56 (m, 2'-$CH_2$ plus $(CD_3)_2SO$), 4.58 (3H, m, 5'-$CH_2$,4'-CH), 5.69 (1H, m, 3'-CH), 6.35 (1H, m, 1'-CH), 6.73 (1H, d, J=13.5 Hz, CH=CHBr), 7.32 (1H, d, J=13.5 Hz, CH=CHBr), 7.48-8.2 (9H, m, 2x$C_6H_4$, 6-CH), 11.77 (1H, s, $D_2O$ exchangeable, NH).

Analysis

Found: C, 49.22; H, 3.10; N, 4.47%. $C_{25}H_{19}BrCl_2N_2O_7$ requires: C, 49.21; H, 3.14; N, 4.59%.

Example 16

E-5-(2-Bromovinyl)-3',5'-di-O-p-methoxybenzoyl-2'-deoxyuridine

E-5-(2-Bromovinyl)-2'-deoxyuridine (1.23 g, 3.7 mmol) was dissolved in dry pyridine (15 mL, cooled in ice and treated with p-anisoyl chloride (1.6 mL, 12 mmol). The solution was stirred at room temperature overnight, poured into ice/water (300 mL) and extracted with three 50 mL portions of ethyl acetate. The combined organic phase was washed with two 25 mL portions of 5N hydrochloric acid, two 25 mL portions of saturated sodium carbonate and 25 mL of distilled water. After drying over magnesium sulphate the solvent was removed and the residue columned on silica eluting with ethyl acetate/cyclohexane (1:1) to give two fractions. The first fraction required further chromatography eluting with ethyl acetate/cyclohexane (1:4) to give a pure sample of E-5-(2-bromovinyl)-3-N-p-methoxybenzoyl-3',5'-di-O-p-methoxybenzoyl-2'-deoxyuridine (0.35 g, 12%), properties reported above in Example 14.

The second fraction proved to be E-5-(2-bromovinyl)-3',5'-di-O-p-methoxybenzoyl-2'-deoxyuridine (1.2 g, 54%), recrystallised from ethyl acetate, mp 204-5°C.

Rf = 0.23 in ethyl acetate/cyclohexane (1:1).
$\nu_{max.}$ (KBr) 3320 (NH), 1730, 1710, 1690 (C = O) $cm^{-1}$
$^1$Hnmr [$(CD_3)_2SO$] $\delta$ 2.58 (m, 2'-$CH_2$ plus $(CD_3)_2SO$), 3.88 (6H, s, 2x$CH_3O$), 4.58 (3H, m, 5'-$CH_2$, 4'-CH), 5.64 (1H, m, 3'-CH), 6.33 (1H, m, 1'-CH), 6.69 (1H, d, J=13.5 Hz, CH=CHBr), 6.90-8.10 (10H, m, 2x$C_6H_4$, 6-CH, CH=CHBr), 11.64 (1H,s, $D_2O$ exchangeable, NH).

Analysis

Found: C, 54.23; H, 4.27; N, 4.72% $C_{27}H_{25}BrN_2O_9$ requires: C, 53.92; H, 4.19; N, 4.66%.

Example 17

E-5-(2-Bromovinyl)-3',5'-di-O-phenylacetyl-2'-deoxyuridine

The title product was prepared by the method described for Example 1 using E-5-(2-bromovinyl)-2'-deoxyuridine (2.15 g, 6.4 mmol), dry pyridine (50 mL) and phenylacetyl chloride (3.4 mL, 26 mmol). Column chromatography was carried out on silica eluting with ethyl acetate/cyclohexane (1:3) to give 2 g of crude material. After further chromatography 0.5 g (14%) of pure E-5-(2-bromovinyl)-3',5'-di-O-phenylacetyl-2'-deoxyuridine was obtained and recrystallised from ethanol, mp 118-19°C,

Rf = 0.35 in ethyl acetate/cyclohexane (1:1).
$\nu_{max}$ (KBr) 3330-2800 (NH), 1735, 1710, 1680 (C=O) $cm^{-1}$
$^1$Hnmr ($CDCl_3$) $\delta$ 1.98 (2H, m, 2'-$CH_2$), 3.67 (4H, s, 2x$CH_2$), 4.23 (1H, m, 4'-CH), 4.40 (2H, m, 5'-$CH_2$), 5.1 (1H, m, 3'-CH), 6.12 (1H, m, 1'-CH), 6.72 (1H,d, J=13.5 Hz, CH=CHBr), 7.1-7.6 (12H, m, 2x$C_6H_5$, 6-CH, CH=CHBr), 9.50 (1H, s, $D_2O$ exchangeable, NH).

Analysis

Found: C, 57.05; H, 4.42; N, 4.84 % $C_{27}H_{25}BrN_2O_7$ requires: C, 56.95; H, 4.42; N, 4.92 %.

Example 18

E-5-(2-Bromovinyl)-5'-O-p-chlorobenzoyl-2'-deoxyuridine

The title product was prepared as described for Example 1 using E-5-(2-bromovinyl)-2'-deoxyuridine (1.06 g, 3.2 mmol), dry pyridine (15 mL), and p-chlorobenzoyl chloride (0.44 mL, 3.5 mmol), stirred at room temperature for 1.45 hours. After recrystallisation from ethanol/water, 0.76 g (50%) of E-5-(2-bromovinyl)-5'-O-p-chlorobenzoyl-2'-deoxyuridine was obtained, mp 209-10°C,

Rf = 0.53 in chloroform/ethanol (10:1)

$\nu_{max.}$ (KBr), 3500-2900 (NH, OH), 1720, 1660 (C=O) $cm^{-1}$

$^1$Hnmr [$(CD_3)_2SO$] $\delta$ 2.29 (2H, m, 2'-$CH_2$), 4.03 (1H, m, 4'-CH), 4.54 (3H, m, 5'-$CH_2$, 3'-CH), 5.50 (1H, d, J=4.5 Hz, $D_2O$ exchangeable, 3'-OH), 6.24 (1H, m, 1'-CH), 6.74 (1H, d, J=13.5 Hz, CH=CHBr), 7.29 (1H, d, J=13.5 Hz, CH=CHBr), 7.5-8.3 (5H, m, $C_6H_4$, 6-CH), 11.78 (1H, s, $D_2O$ exchangeable, NH).

Analysis

Found: C, 45.83; H, 3.33; N, 5.66% $C_{18}H_{16}BrClN_2O_6$ requires: C, 45.83; H, 3.42; N, 5.94%.

Example 19

E-5-(2-Bromovinyl)-5'-p-methoxybenzoyl-2'-deoxyuridine

The title product was prepared as described for Example 1 using E-5-(2-bromovinyl)-2'-deoxyuridine (1.19 g, 3.6 mmol) in dry pyridine (15 mL) and p-anisoyl chloride (0.53 mL, 3.9 mmol), stirred at room temperature for 3 hours. After recrystallisation from ethanol/water, 0.4 g (24%) of E-5-(2-bromovinyl)-5'-p-methoxybenzoyl-2'-deoxyuridine was obtained, mp 190-1°C,
Rf = 0.51 in chloroform/ethanol (10:1)
$\nu_{max}$ 3500-2800 (NH, OH), 1725, 1700, 1655 (C=O) $cm^{-1}$
$^1$Hnmr [$(CD_3)_2SO$] $\delta$ 2.27 (2H, m, 2'-$CH_2$), 3.87 (3H, s, $CH_3O$), 4.08 (1H, m, 4'-CH), 4.47 (3H, m, 5'-$CH_2$, 3'-CH), 5.50 (1H, d, J=4.5 Hz, $D_2O$ exchangeable, 3'-OH), 6.24 (1H, m, 1'-CH), 6.70 (1H, d, J=13.5 Hz, CH=CHBr), 7.0-8.1 (6H, m, $C_6H_4$, 6-CH, CH=CHBr), 11.66 (1H, s, $D_2O$ exchangeable, NH).

Analysis

Found: C, 48.54; H, 4.22; N, 5.95 % $C_{19}H_{19}BrN_2O_7$ requires: C, 48.84; H, 4.10; N, 6.00%.

Example 20

E-5-(2-Bromovinyl)-3-N-p-chlorobenzoyl-2'-deoxyuridine

The title product was prepared by the method described for Example 12 using E-5-(2-bromovinyl)-3-N-p-chlorobenzoyl-3',5'-di-O-p-chlorobenzoyl-2'-deoxyuridine (0.75 g, 1 mmol) in 0.02 sodium methoxide in methanol (60 mL, 1.2 mmol) stirred at room temperature for 10 minutes. After column chromatography on silica eluting with chloroform/ethanol (20:1) 0.3 g (61%), of E-5-(2-bromovinyl)-3-N-p-chlorobenzoyl-2'-deoxyuridine was obtained and recrystallised from ethanol/water, mp 189-90°C,

Rf = 0.38 in chloroform/ethanol (10:1)

$\nu_{max}$ 3600-2900 (OH), 1755, 1700, 1660 (C=O) $cm^{-1}$

$^1$Hnmr [$(CD_3)_2SO$] $\delta$ 2.25 (2H, m, 2'-$CH_2$), 3.67 (2H, m, 5'-$CH_2$), 3.80 (1H, m, 4'-CH), 4.30 (1H, m, 3'-CH) 5.18 (1H, t, J=5 Hz, $D_2O$ exchangeable, 5'-OH), 5.29 (1H, d, J=4 Hz, $D_2O$ exchangeable, 3'-OH), 6.11 (1H, m, 1'-CH), 6.94 (1H, d, J=13.5 Hz, CH=CHBr), 7.23 (1H, d, J=13.5 Hz, CH=CHBr), 7.5-8.3 (4H, m, $C_6H_4$), 8.42 (1H, s, 6-CH).

Analysis

Found: C, 46.02; H, 3.67; N, 5.90 % $C_{18}H_{16}BrClN_2O_6$ requires: C, 45.83; H, 3.42; N, 5.94%.

Example 21

E-5-(2-Bromovinyl)-3-N-p-methoxybenzoyl-2'-deoxyuridine

The title product was prepared by the method described for Example 12 using E-5-(2-bromovinyl)-3-N-p-methoxybenzoyl-3',5'-di-O-p-methoxybenzoyl-2'-deoxyuridine (0.74 g, 1 mmol) in 0.02 sodium methoxide in methanol stirred at room temperature for 4.5 hours. After column chromatography on silica eluting with chloroform/ethanol (20:1) 0.3 g (64%) E-5-(2-bromovinyl)-3-N-p-methoxybenzoyl-2'-deoxyuridine was obtained and recrystallised from ethanol/water, mp 172-3°C,

Rf = 0.41 in chloroform/ethanol (10:1)

$\nu_{max}$ 3100-2900 (OH), 1740, 1700, 1680 (C=O) $cm^{-1}$

$^1$Hnmr [$(CD_3)_2SO$] δ 2.28 (2H, m, 2'-$CH_2$), 3.70 (2H, m, 5'-$CH_2$), 3.88 (4H, m, $CH_3O$, 4'-CH), 4.30 (1H, m, 3'-CH) 5.14 (1H, t, J=5 Hz, $D_2O$ exchangeable, 5'-OH), 5.27 (1H, d, J=4 Hz, $D_2O$ exchangeable, 3'-OH), 6.14 (1H, m, 1'-CH), 6.7-8.2 (6H, m, $C_6H_4$, CH=CHBr, CH=CHBr), 8.35 (1H, s, 1'-CH).

Analysis

Found: C, 49.01; H, 3.92; N, 5.91 % $C_{19}H_{19}BrN_2O_7$ requires: C, 48.84; H, 4.10; N, 6.00%.

Example 22

E-5-(2-Bromovinyl)-3',5'-bis-O-trimethylsilyl-2'-deoxyuridine

N,O-*bis*Trimethylsilylacetamide (1.5 mL) was added to a stirred solution of *E*-5-(2-bromovinyl)-2'-deoxyuridine (1.0 g) in tetrahydrofuran (50 mL) at room temperature. The mixture was then stirred at room temperature for 0.5 hours and then evaporated under reduced pressure to give the required bis-trimethylsilylated product (1.4 g), mp 137-140°C, $\nu_{max}$ (EtOH) 250 ($\epsilon$ 14,300) and 292 ($\epsilon$ 12,500) nm, $\lambda_{max}$ (KBr) 1705, 1465, 1252, 1118 and 842 $cm^{-1}$, $^1$Hnmr ($CDCl_3$) $\delta$ 0.14 (9H, s, $Si(CH_3)_3$), 0.23 (9H, s, $Si(CH_3)_3$), 1.89-2.48 (2H, m, 2'-$CH_2$), 3.61-3.95 (2H, m, 5'-$CH_2$), 3.97 (1H, m, 4'-CH), 4.40 (1H, m, 3'-CH), 6.32 (1H, t, J=6 Hz, 1'-CH), 6.70 (1H, d, J=14 Hz, CH=CHBr), 7.43 (1H, d, J=14 Hz, CH=CHBr), 7.93 (1H, s, 6-CH), 9.36 (1H, m, 3-NH), M.s. observed 476.0835 ($M^+$ theoretical 476.0798).

Analysis

Found: C, 42.2; H, 6.15; N, 5.8 % $C_{17}H_{29}BrN_2O_5Si_2$ requires: C, 42.75; H, 6.1; N, 5.85%

Example 23

E-5-(2-Bromovinyl)-3-N-valeryl-2'-deoxyuridine

To a stirred solution of E-5-(2-bromovinyl)-3',5'-bis-O-trimethylsilyl-2'-deoxyuridine (Example 22) (1.0 g) in tetrahydrofuran (20 mL) at -20°, triethylamine (0.45 mL) was added followed by valeryl chloride (0.28 mL). The mixture was stirred at -20° for 0.5 hours and then partitioned between water and ethyl acetate at pH 1 by addition of aqueous hydrochloric acid. The organic layer was washed with water and aqueous sodium bicarbonate, dried and evaporated. The residue was adsorbed on silica gel (6 g) and chromatographed on silica gel (40 g). Elution of the column with n-hexane-ethyl acetate (1:4) gave the required 3-valeryl derivative (0.25 g, 29%) as an oil.

$\lambda_{max.}$ (EtOH) 250 ($\varepsilon$ 14,100) and 297 ($\varepsilon$ 10,300) nm, $\nu_{max.}$ ($CHCl_3$) 1795, 1710, 1670, 1455, 1295, 1100 $cm^{-1}$, $^1$Hnmr ($CDCl_3$) $\delta$ 0.94 (3H, broad, J=7 Hz, $CH_3$), 1.20-1.90 (4H, m, $(CH_2)_2$), 2.10-2.50 (4H, m, 2'-$CH_2$, 3'-OH, 5'-OH), 2.83 (2H, t, J=7.5 Hz, $CH_2CO$), 3.86-4.06 (3H, m, 5'-$CH_2$, 4'-CH), 4.56 (1H, m, 3'-CH), 6.22 (1H, t, J=6 Hz, 1'-CH), 6.65 (1H, d, J=14 Hz, CH=CHBr), 7.31 (1H, d, J=14 Hz, CH=CHBr), 7.86 (1H, s, 6-CH), M.s. observed 416.0552 ($M^+$ theoretical 416.0579).

Example 24

E-5-(2-Bromovinyl)-3-N-pivaloyl-2'-deoxyuridine

To a stirred solution of E-5-(2-bromovinyl)-3',5'-bis-O-trimethylsilyl-2'-deoxyuridine (o.6 g) in tetrahydrofuran (20 mL) at 0°, triethylamine (0.35 mL) and 4-dimethylaminopyridine (0.01 g) were added followed by pivaloyl chloride (0.23 mL). The mixture was stirred at 0° for 10 minutes and then partitioned between water and ethyl acetate at pH 1 by addition of aqueous hydrochloric acid. The organic layer was washed with water and aqueous sodium bicarbonate, dried and evaporated, affording an oil which was chromatographed on silica gel (25 g). Elution with n-hexane-ethyl acetate (1:4) gave the required 3-pivaloyl derivative (0.46 g, 88%) as a glass. $\lambda_{max.}$ (EtOH) 250 ($\varepsilon$ 14,300) and 297 ($\varepsilon$ 10,500) nm, $\nu_{max.}$ ($CHCl_3$) 1780, 1705, 1665, 1455, 1295, 1095 $cm^{-1}$; $^1$Hnmr ($CDCl_3$-DMSO-$d_6$) $\delta$ 1.34 (9H, s, $(CH_3)_3$), 2.06-2.62 (2H, m, 2'-$CH_2$), 3.83 (2H, m, 5'-$CH_2$), 4.00 (1H, m, 4'-CH), 4.46 (1H, m, 3'-CH), 4.86 (2H, m, 3'-OH, 5'-OH), 6.30 (1H, t, J=6 Hz, 1'-CH), 6.81 (1H, d, J=14 Hz, CH=CHBr), 7.33 (1H, d, J=14 Hz, CH=CHBr), 8.38 (1H, s, 6-CH), M.s. observed 416.0542 ($M^+$ theoretical 416.0582).

0082668

Example 25

E-5-(2-Bromovinyl)-3-N-cyclohexanecarbonyl-2'-deoxyuridine

To a stirred solution of E-5-(2-bromovinyl)-3',5'-bis-O-trimethylsilyl-2'-deoxyuridine (0.6 g) in tetrahydrofuran (15 mL), 4-dimethylaminopyridine (0.01 g) and triethylamine (0.26 mL) was added, followed by cyclohexanecarboxylic acid chloride (0.18 mL). The mixture was stirred at room temperature overnight and then partitioned between water and ethyl acetate at pH 1 by addition of aqueous hydrochloric acid. The organic layer was washed with water and aqueous sodium bicarbonate, dried and evaporated, affording a gum which was adsorbed on silica gel (3 g) and then chromatographed on silica gel (20 g). Elution of the column with n-hexane-ethyl acetate (1:4) gave the required 3-cyclohexanecarbonyl derivative (0.19 g, 34%) as a glass,

$\lambda_{max.}$ (EtOH) 250 ($\varepsilon$ 14,300) and 297 ($\varepsilon$ 10,100) nm, $\nu_{max.}$ ($CHCl_3$) 1785, 1705, 1665, 1455, 1290, 1095 $cm^{-1}$; $^1H$ nmr ($CDCl_3$) $\delta$ 1.12-2.20 (10H, m, $(CH_2)_5$), 2.34 (2H, m, 2'-$CH_2$), 2.64 (2H, m, 3'-OH, 5'-OH), 2.75 (1H, m, cyclohexyl-CH), 3.84-4.10 (3H, m, 5'-$CH_2$, 4'-CH), 4.53 (1H, m, 3'-CH), 6.20 (1H, t, J=6 Hz, 1'-CH), 6.68 (1H, d, J=14 Hz, CH=CHBr), 7.35 (1H, d, J=14 Hz, CH=CHBr), 7.95 (1H, s, 6-CH), M.s. observed 442.0735 ($M^+$ theoretical 442.0736).

0082668

Example 26

E-5-(2-Bromovinyl)-3-N-(3-chlorobenzoyl)-2'-deoxyuridine

To a stirred solution of E-5-(2-bromovinyl)-3',5'-bis-O-trimethylsilyl-2'-deoxyuridine (0.6 g) in tetrahydrofuran (15 mL) at 0°, triethylamine (0.5 mL) and 4-dimethylaminopyridine (0.01 g) were added followed by 3-chlorobenzoyl chloride (10.3 mL). The mixture was stirred at 0° for 1.5 hours and then partitioned between water and ethyl acetate at pH 1 by addition of aqueous hydrochloric acid. The organic layer was washed with water and aqueous sodium bicarbonate, dried and evaporated, affording a solid which was adsorbed on silica gel (3 g) and then chromatographed on silica gel (20 g). Elution of the column with n-hexane-ethyl acetate (1:4) gave the required 3-(3-chlorobenzoyl) derivative (0.45 g, 76%) as needles, mp 178-182°C (from ethanol-water),

$\lambda_{max}$ (EtOH) 248 ($\varepsilon$ 25,600) and 298 ($\varepsilon$ 11,000) nm, $\nu_{max}$ (KBr) 1760, 1700, 1640, 1450, 1280 $cm^{-1}$, $^1$Hnmr (DMSO-$d_6$) $\delta$ 2.25 (2H, m, 2'-$CH_2$), 3.66 (2H, m, 5'-$CH_2$), 3.83 (1H, m, 4'-CH), 4.30 (1H, m, 3'-CH), 5.10-5.35 (2H, m, 5'-OH, 3'-OH), 6.12 (1H, t, J=6 Hz, 1'-CH), 6.96 (1H, d, J=14 Hz, CH=CHBr), 7.26 (1H, d, J=14 Hz, CH=CHBr), 7.56-8.25 (4H, m, aromatic), 8.37 (1H, s, 6-CH),

Analysis

Found: C, 46.15; H, 3.6; N, 5.75% $C_{18}H_{16}BrClN_2O_6$ requires: C, 45.85; H, 3.4; N, 5.95%.

Example 27

E-5-(2-Bromovinyl)-3-[4-(N,N-di-n-propylsulphamoyl)-benzoyl]-3',5'-di-O-[4-(N,N-di-n-propylsulphamoyl)-benzoyl-2'-deoxyuridine

and

Example 28

E-5-(2-Bromovinyl)-3',5'-di-O-([4-(N,N-di-n-propyl-sulphamoyl)benzoyl]-2'-deoxyuridine

(a) 4-(N,N-Di-propylsulphamoyl)benzoylchloride

A suspension of 4-(N,N-di-*n*-propylsulphamoyl) benzoic acid (4.12 g, 14 mmol) and oxaloyl chloride (7.5 mL, 87 mmol) in dry toluene (30 mL) was boiled under reflux for 3 hours. The solvent was removed and the residue subjected to high vacuum for 2 hours, then triturated with a small volume of cold cyclohexane. A white solid resulted, and was filtered under nitrogen, ground with a pestle and washed with cyclohexane. Residual cyclohexane was removed under reduced pressure to give 4-(N,N-di-*n*-propylsulphamoyl)benzoyl chloride (3.6 g, 82%) which was used without further purification,

$\nu_{max}$ (KBr) 1795, 1780, 1745 (C=O) $cm^{-1}$,
$^1$Hnmr ($CDCl_3$) $\delta$ 0.88 (6H, t, J=6Hz, 2x$CH_3$), 1.64 (4H, t q, $J_t$ = 7.5Hz, $J_q$ = 6Hz, 2x$CH_2$), 3.07 (4H, t, J =7.5Hz, 2x$CH_2N$), 8.04 (4H, m, $C_6H_4$).

(b) A solution of *E*-5-(2-bromovinyl)-2'-deoxyuridine (0.36 g, 1 mmol), 4-(N,N-di-*n*- dipropylsulphamoyl) benzoyl chloride (1.3 g, 4.2 mmol) and dimethylaminopyridine (0.1 g) in dry pyridine (10 mL) was stirred at room temperature for 18 hours, then poured into

ice/water (300 mL) and extracted with ethyl acetate (3 x 50 mL). The ethyl acetate solutions were washed with 5N hydrochloric acid (100 mL), saturated sodium carbonate solution (2 x 25 ml) and water (100 mL) and then dried (magnesium sulphate) and evaporated under reduced pressure. The residue was column chromatographed on silica, eluting with ethyl acetate/cyclohexane (1:2). The first component to elute was the tri-acylated derivative (Example 27) (0.56 g, 50%), recrystallised from ethanol, mp 102-104°C;

Rf 0.52 (ethyl acetate/cyclohexane 1:1);
$\nu_{max}$ (KBr) 1755, 1730, 1710, 1670 (C=O) $cm^{-1}$;
$^1H$ nmr ($CDCl_3$) $\delta$ 0.88 (18H, t, J=6Hz, 6 x $CH_3$), 1.54 (12H, t q, $J_t$ = 7.5Hz, $J_q$ = 6.0Hz, 6 x $CH_2N$), 2.64 (2H, m, 2'-$CH_2$), 3.13 (12H, t, J = 7.5Hz, 6 x $CH_2N$), 4.63 (1H, m, 4'-CH), 4.80 (2H, m, 5'-$CH_2$), 5.68 (1H, m, 3'-CH), 6.36 (1H, m, 1'-CH), 6.57 (1H, d, J = 13.5Hz, <u>CH</u>=CHBr), 7.20-8.35 (14H, m, 3 x $C_6H_4$, 6-CH, CH=<u>CH</u>Br).

<u>Analysis</u>

Found: C, 53.00; H, 5.70; N, 6.13% $C_{50}H_{64}BrN_5O_{14}S_3$ requires: C, 52.90; H, 5.68; N, 6.17%.

(c) The second component to elute was the di-ester (Example 28) (0.34 g, 39%), recrystallised from ethanol, mp 99-101°C;

Rf 0.31 (ethyl acetate/cyclohexane 1:1);
$\nu_{max}$ (KBr) 3300-2800 (NH), 1730, 1690 (C=O) $cm^{-1}$;
$^1H$ nmr ($CDCl_3$) $\delta$ 0.88 (12H, t, J = 6Hz, 4 x $CH_3$), 1.50 (8H, t q, $J_t$ = 7.5 Hz, $J_q$ = 6Hz, 4 x $CH_2$), 2.58 (2H, m, 2'-$CH_2$), 3.08 (8H, t, J = 7.5Hz, 4 x $CH_2N$), 4.58 (1H, m, 4'-CH), 4.77 (2H, m, 5'-$CH_2$), 5.62 (1H, m, 3'-CH), 6.34 (1H, m, 1'-CH), 6.50 (1H, d, J = 13.5Hz, CH=CHBr), 7.23-8.33 (10H, m, 2 x $C_6H_4$, 6-CH, CH=CHBr), 9.13 (1H, s, $D_2O$ exchangeable NH).

Analysis

Found: C, 51.06; H, 5.44; N, 6.31% $C_{37}H_{47}BrN_4O_{11}S_2$
requires: C, 51.21; H, 5.46; N, 6.46%.

The yield of the tri-acylated derivative (Example 27) was increased to 75% by reaction of E-5-(2-bromovinyl)-2'-deoxyuridine (0.52 g, 1.6 mmol) with 4-(N,N-di-n-propylsulphamoyl)benzoyl chloride (1.9 g, 6.3 mmol) and triethylamine (0.87 mL, 6.3 mmol) in dioxane (10 mL), stirred at room temperature for 18 hours. Work up was carried out as described above to give Example 27 (1.33 g, 75%).

Example 29

E-5-(2-Bromovinyl)-5'-p-(dipropylsulphamoyl)benzoyl-2'-deoxyuridine

A solution of E-5-(2-bromovinyl)-2'-deoxyuridine (1.6 g, 4.8 mmol) and p-(dipropylsulphamoyl)benzoyl chloride (1.5 g, 5 mmol) in dry pyridine (10 mL) was stirred at room temperature for 2 h. The mixture was poured into brine/ice (200 mL) and extracted with chloroform (3 x 50 mL). The combined organic extracts were then treated as described for Examples 27 and 28 and the product column chromatographed on silica, eluting with ethyl acetate/ethanol (50:1), to give the title compound (0.48 g, 17%), recrystallised from ethanol/water, mp 160-161°C;

Rf 0.41 (ethyl acetate);
$\nu_{max}$(KBr) 3500-2700 (NH,OH), 1735, 1710, 1690 (C=O)$cm^{-1}$,
$^1H$ nmr [$(CD_3)_2SO$] $\delta$ 0.81 (6H, t, J=6Hz, 2 x $CH_3$), 1.42 (4H, t q, $J_t$=7.5Hz, $J_q$=6Hz, 2 x $CH_2$), 2.27 (2H, m, 2'-$CH_2$), 3.07 (4H, t, J=7.5Hz, 2 x $CH_2N$), 4.10 (1H, m, 4'-CH), 4.45 (3H, m, 5'-$CH_2$, 3'-CH), 5.50 (1H, s, $D_2O$ exchangeable, 3'-OH), 6.21 (1H, m, 1'-CH), 6.86 (1H, d, J=13.5Hz, CH=CHBr), 7.30 (1H, d, J=13.5Hz, CH=CHBr), 7.8-8.3 (5H, m, $C_6H_4$, 6-CH), 11.37 (1H, s, $D_2O$ exchangeable, NH).

Analysis

Found: C,48.38; H, 4.63; N, 6.85% $C_{24}H_{30}BrN_3O_8S$
requires: C,48.00; H, 5.04; N, 7.00%

0082668

Example 30

E-5-(2-Bromovinyl)-3-[4-(N,N-di-n-propylsulphamoyl)benzoyl]-2'-deoxyuridine

*E*-5-(2-Bromovinyl)-3-[4-(N,N-di-*n*-propylsulphamoyl)benzoyl]-3',5'-di-O-[4-(N,N-di-*n*-propylsulphamoyl)benzoyl]-2'-deoxyuridine (Example 27) (1.0g, 0.9mmol) was dissolved in boiling methanol and the solution cooled to room temperature. The suspension thus formed was treated with 0.02M sodium methoxide in methanol (60mL, 1.2mmol) for 1.5 minutes and then neutralised with Amberlite IR 20(H) resin, filtered, the solvent removed and the residue column chromatographed on silica, eluting with ethyl acetate/cyclohexane (5:1) to give *E*-5-(2-bromovinyl)-3-[4-(N,N-di-*n*-propylsulphamoyl)benzoyl]-2'-deoxyuridine (0.12g, 32%), recrystallised from ethanol/water, m.p. 135-136$^{o}$C; Rf 0.35 (ethyl acetate); νmax. (KBr) 3500-2800 (OH), 1755, 1705, 1665 (C=O) $cm^{-1}$; $^{1}$Hnmr [$(CD_3)_2SO$] δ 0.82 (6H, t, J = 6Hz, 2 x $CH_3$), 1.48 (4H, t q, $J_t$ = 7.5Hz, $J_q$ = 6Hz, 2 x $CH_2$), 2.23 (2H, m, 2'-$CH_2$), 3.07 (4H, t, J = 7.5Hz, 2 x $CH_2N$), 3.68 (2H, m, 5'-$CH_2$), 3.83 (1H, m, 4'-CH), 4.30 (1H, m, 3'-CH), 5.17 (1H, t, J = 6Hz, $D_2O$ exchangeable, 5'-OH), 5.26 (1H, d, J = 4Hz, $D_2O$ exchangeable, 3'-OH), 6.12 (1H, m, 1'-CH), 6.94 (1H, d, J = 13.5Hz, C<u>H</u>=CHBr), 7.23 (1H, d, J = 13.5Hz, CH=C<u>H</u>Br), 7.9-8.5 (5H, m, $C_6H_4$, 6-CH).

Analysis

Found: C, 48.02; H, 5.10; N, 6.88 %. $C_{24}H_{30}BrN_3O_8S$ requires C, 48.00; H, 5.04; N, 7.00 %.

Example 31

E-5-(2-bromovinyl)-3',5'-di-O-(4-nitrobenzoyl)-2'-deoxyuridine

To a solution of E-5-(2-bromovinyl)-2'-deoxyuridine (0.73g, 2.2mmol) in dry pyridine (10mL) cooled in ice was added p-nitrobenzoyl chloride (0.89g, 4.8mmol). After stirring at room temperature for 18 hours the mixture was poured into ice/water (200mL) and the resulting precipitate filtered, washed with water and recrystallised from acetone to give E-5-(2-bromovinyl)-3',5'-di-O-(4-nitrobenzoyl)-2'-deoxyuridine (0.71g, 51%), m.p. 244-245°C (dec), Rf 0.27 (ethyl acetate/cyclohexane 1:1); νmax (KBr) 3295, 3110, 3080 (NH), 1730, 1710, 1685 (C=O) $cm^{-1}$; $^1$Hnmr [$(CD_3)_2SO$] δ 2.74 (2H, m, 2'-$CH_2$, $(CD_3)_2SO$), 4.70 (3H, m, 5'-$CH_2$, 4'-CH), 5.78 (1H, m, 3'-CH), 6.35 (1H, m, 1'-CH), 6.66 (1H, d, J = 13.5Hz, CH=CHBr), 7.25 (1H, d, J = 13.5Hz, CH=CHBr), 7.8-8.7 (9H, m, 2 x $C_6H_4$, 6-CH), 11.67 (1H, s, $D_2O$ exchangeable, NH).

Analysis

Found: C, 47.33; H, 2.77; N, 8.70 %. $C_{25}H_{19}BrN_4O_{11}$ requires C, 47.56; H, 3.03; N, 8.87 %.

Example 31

E-5-(2-Bromovinyl)-5'-O-(4-nitrobenzoyl)-2'-deoxyuridine

To a solution of E-5-(2-bromovinyl)-2'-deoxyuridine (2.08g, 6.2mmol) in dry N,N-dimethylformamide (20mL) cooled in ice was added 4-dimethylaminopyridine (0.69g, 5.6mmol) and p-nitrobenzoyl chloride (1.04g, 5.6mmol). The solution was stirred at room temperature for three hours and then poured into ice/water (200mL). The solid product was columned on silica eluting with ethyl acetate/cyclohexane 5:1, increasing polarity to ethyl acetate/cyclohexane 20:1. After elution of E-5-(2-bromovinyl)-3',5'-di-O-(4-nitrobenzoyl)-2'-deoxyuridine (0.5g, 13%) the major product E-5-(2-bromovinyl)-5'-(4-nitrobenzoyl)-2'-deoxyuridine was collected (1g, 33%) and recrystallised from acetone, m.p. 220-221$^{o}$C (dec); Rf 0.30 (chloroform/ethanol 20:1); νmax (KBr) 3100-2900 (NH, OH), 1730, 1715, 1680, 1660 (C=O) $cm^{-1}$; $^{1}$Hnmr [$(CD_3)_2SO$] δ 2.30 (2H, m, 2'-$CH_2$), 4.10 (1H, m, 4'-CH), 4.55 (3H, m, 5'-$CH_2$, 3'-CH), 5.50 (1H, d, J = 3Hz, $D_2O$ exchangeable, 3'-OH), 6.21 (1H, m, 1'-CH), 6.67 (1H, d, J = 13.5Hz, CH=CHBr), 7.26 (1H, d, J = 13.5Hz, CH=CHBr), 7.74-8.50 (5H, m, $C_6H_4$, 6-CH), 11.63 (1H, s, $D_2O$ exchangeable, NH).

Analysis

Found: C, 44.92; H, 2.91; N, 8.50 %. $C_{18}H_{16}BrN_3O_8$ requires C, 44.83; H, 3.34; N, 8.71 %.

<u>General Method for the Preparation of 3',5'-di-O-Aroyl, 5'-O-Aroyl and 3'-O-Aroyl Esters of E-5-(2-bromovinyl)-2'-deoxyuridine (Examples 33-43)</u>

To a solution of E-5-(2-bromovinyl)-2'-deoxyuridine (2g, 6mmol) in dry N,N-dimethylformamide (20mL), cooled in ice, was added 4-dimethylaminopyridine (8.4mmol) and the appropriate aroyl chloride (8.4mmol). The mixture was stirred at room temperature for a period varying between 3 and 22 hours, poured into brine/ice (200mL) and the resulting solid columned on silica to give the aroyl esters Examples 33-43.

Examples 33, 34 and 35

E-5-(2-Bromovinyl)-3',5'-di-O-(3,4-dichlorobenzoyl)-2'-deoxyuridine, E-5-(2-Bromovinyl)-3'-O-(3,4-dichlorobenzoyl)-2'-deoxyuridine and E-5-(2-Bromovinyl)-5'-O-(3,4-dichlorobenzoyl)-2'-deoxyuridine

Reaction of E-5-(2-bromovinyl)-2'-deoxyuridine with 3,4-dichlorobenzoyl chloride for 3 hours gave a mixture of products which were separated by column chromatography on silica eluting initially with chloroform and increasing polarity to chloroform/ethanol 20:1. The first compound to elute was E-5-(2-bromovinyl)-3',5'-di-O-(3,4-dichlorobenzoyl)-2'-deoxyuridine (Example 33) (27%), recrystallised from carbon tetrachloride, m.p. 171-172°C; Rf 0.53 (ethyl acetate/cyclohexane 1:1); νmax (KBr) 3300 (NH), 1730, 1690 (C=O) $cm^{-1}$; $^1$Hnmr ($CDCl_3$) δ 2.55 (2H, m, 2'-$CH_2$), 4.55 (1H, m, 4'-CH), 4.73 (2H, m, 5'-$CH_2$), 5.60 (1H, m, 3'-CH), 6.40 (2H, m, 1'-CH, CH=CHBr), 7.2-8.2 (8H, m, 2 x $C_6H_3$, 6-CH, CH=CHBr), 9.03 (1H, s, $D_2O$ exchangeable, NH).

Analysis

Found: C, 44.14; H, 2.20; N, 4.08 %. $C_{25}H_{17}BrCl_4N_2O_7$ requires C, 44.21; H, 2.25; N, 4.12 %.

The second component to elute was E-5-(2-bromovinyl)-3'-O-(3,4-dichlorobenzoyl)-2'-deoxyuridine (Example 34) (3%), recrystallised from ethyl acetate, m.p. 217-218°C; Rf 0.38 (ethyl acetate); νmax (KBr) 3200-2800 (NH, OH), 1725, 1705, 1685 (C=O) $cm^{-1}$; $^1$Hnmr [$(CD_3)_2SO$] δ 2.50 (2H, m, 2'-$CH_2$, $(CD_3)_2SO$), 3.74 (2H, m, 5'-$CH_2$), 4.25 (1H, m, 4'-CH), 5.30 (1H, m, $D_2O$ exchangeable, 5'-OH), 5.50 (1H, m, 3'-CH), 6.89 (1H, d, J = 13.5Hz, CH=CHBr), 7.29 (1H, d, J = 13.5Hz, CH=CHBr), 7.7-8.3 (5H, m, $C_6H_4$, 6-CH), 11.60 (1H, s, $D_2O$ exchangeable, NH).

Analysis

Found: C, 42.94; H, 2.76; N, 5.35 %. $C_{18}H_{15}BrCl_2N_2O_6$ requires C, 42.71; H, 2.99; N, 5.53 %.

Cont'd./....

The final component to elute was *E*-5-(2-bromovinyl)-5'-O-(3,4-dichlorobenzoyl)-2'-deoxyuridine (Example 35) (26%), recrystallised from ethyl acetate, m.p. 207-208°C (dec); Rf 0.32 (chloroform/ethanol 20:1); νmax (KBr) 3600-3000 (NH,OH), 1720, 1680, 1660 (C=O) $cm^{-1}$; $^{1}H$nmr [$(CD_3)_2SO$] δ 2.29 (2H, m, 2'-$CH_2$), 4.10 (1H, m, 4'-CH), 4.49 (3H, m, 5'-$CH_2$, 3'-CH), 5.48 (1H, d, J = 3Hz, 3'-OH, $D_2O$ exchangeable), 6.20 (1H, m, 1'-CH), 6.72 (1H, d, J = 13.4Hz, C<u>H</u>=CHBr), 7.24 (1H, d, J = 13.5Hz, CH=C<u>H</u>Br), 7.7-8.2 (4H, m, $C_6H_3$, 6-CH), 11.58 (1H, s, $D_2O$ exchangeable NH).

<u>Analysis</u>

Found: C, 42.74; H, 2.59; N, 5.50 %. $C_{18}H_{15}BrCl_2N_2O_6$ requires C, 42.71; H, 2.99; N, 5.53 %.

BAD ORIGINAL

Examples 36, 37 and 38

E-5-(2-Bromovinyl)-3',5'-di-O-(4-methylbenzoyl)-2'-deoxyuridine, E-5-(2-Bromovinyl)-3'-O-(4-methylbenzoyl)-2'-deoxyuridine and E-5-(2-Bromovinyl)-5'-O-(4-methylbenzoyl)-2'-deoxyuridine

Reaction of E-5-(2-bromovinyl)-2'-deoxyuridine with p-methylbenzoyl chloride for 4.5 hours gave a mixture of products which were separated by column chromatography on silica, eluting initially with chloroform and gradually increasing polarity to chloroform/ethanol 20:1. The first component to elute was E-5-(2-bromovinyl)-3',5'-di-O-(4-methylbenzoyl)-2'-deoxyuridine (Example 36) (12%), recrystallised from ethyl acetate, m.p. 205-206$^{o}$C; Rf 0.47 (ethyl acetate/cyclohexane 1:1); νmax (KBr) 3300-2900 (NH), 1730, 1720, 1680 (C=O) $cm^{-1}$; $^{1}$Hnmr [$(CD_3)_2SO$] δ 2.54 (8H, m, 2 x $CH_3$, 2'-$CH_2$, $(CD_3)_2SO$), 4.65 (3H, m, 5'-$CH_2$, 4'-CH), 5.70 (1H, m, 3'-CH), 6.40 (1H, m, 1'-CH), 6.70 (1H, d, J = 13.5Hz, CH=CHBr), 7.2-8.2 (10H, 2 x $C_6H_4$, 6-CH, CH=CHBr), 11.70 (1H, s, $D_2O$ exchangeable NH);

Analysis

Found: C, 56.63; H, 4.32; N, 4.87 %. $C_{27}H_{25}BrN_2O_7$ requires C, 56.95; H, 4.43; N, 4.92%.

The second component to elute was E-5-(2-bromovinyl)-3'-O-(4-methylbenzoyl)-2'-deoxyuridine (Example 37) (4%), recrystallised from ethyl acetate/ cyclohexane, m.p. 206-208$^{o}$C; Rf 0.61 (ethyl acetate); νmax 3600-2900 (NH,OH), 1710, 1685 (C=O) $cm^{-1}$; $^{1}$Hnmr [$(CD_3)_2SO$] δ 2.47 (5H, m, $CH_3$, 2'-$CH_2$, $(CD_3)_2SO$), 3.73 (2H, m, 5'-$CH_2$), 4.20 (1H, m, 4'-CH), 5.29 (1H, m, $D_2O$ exchangeable 5'-OH), 5.50 (1H, m, 3'-CH), 6.29 (1H, m, 1'-CH), 6.89 (1H, d, J = 13.5Hz, CH=CHBr), 7.2-8.2 (6H, m, $C_6H_4$, 6-CH, CH=CHBr), 11.60 (1H, s, $D_2O$ exchangeable, NH).

Analysis

Found: C, 50.64; H, 4.20; N, 5.94 %. $C_{19}H_{19}BrN_2O_6$ requires C, 50.52; H, 4.24; N, 6.21 %.

Cont'd./.....

The final component to elute was E-5-(2-bromovinyl)-5'-O-(4-methylbenzoyl)-2'-deoxyuridine (Example 28) (28%), recrystallised from ethyl acetate, m.p. 219-220°C (dec); Rf 0.31 (chloroform/ethanol 20:1); νmax (KBr) 3100-2900 (NH, OH), 1715, 1690 (C=O) $cm^{-1}$; $^1$Hnmr [$(CD_3)_2SO$] δ 2.38 (5H, m, $CH_3$, 2'-$CH_2$, $(CD_3)_2SO$), 4.10 (1H, m, 4'-CH), 4.50 (3H, m, 5'-$CH_2$, 3'-CH), 5.49 (1H, d, J = 3Hz, $D_2O$ exchangeable, 3'-OH), 6.22 (1H, m, 1'-CH), 6.67 (1H, d, J = 13.5Hz, C<u>H</u>=CHBr), 7.1-8.1 (6H, m, $C_6H_4$, 6-CH, CH=C<u>H</u>Br), 11.60 (1H, s, $D_2O$ exchangeable, NH).

<u>Analysis</u>

Found: C, 50.49; H, 3.88; N, 6.08 %. $C_{19}H_{19}BrN_2O_6$ requires C, 50.52; H, 4.24; N, 6.21 %.

BAD ORIGINAL

Examples 39 and 40

E-5-(2-Bromovinyl)-3',5'-di-O-(4-n-butylbenzoyl)-2'-deoxyuridine and E-5-(2-Bromovinyl)-5'-O-(4-n-butylbenzoyl)-2'-deoxyuridine

Reaction of E-5-(2-bromovinyl)-2'-deoxyuridine with p-n-butylbenzoyl chloride for 4 hours gave a mixture of products which were separated by column chromatography on silica, eluting initially with chloroform and gradually increasing polarity to chloroform/ethanol 20:1. The first component to elute was E-5-(2-bromovinyl)-3',5'-di-O-(4-n-butylbenzoyl)-2'-deoxyuridine (Example 39) (31%), recrystallised from ethyl acetate, m.p. 161-162°C; Rf 0.61 (ethyl acetate/cyclohexane 1:1); νmax (KBr) 3600-2800 (NH), 1730, 1710, 1685 (C=O) $cm^{-1}$; $^1$Hnmr [$(CD_3)_2SO$] δ 0.90 (6H, t, J = 6Hz, 2 x $CH_3$), 1.44 (8H, m, 4 x $CH_2$), 2.64 (6H, m, 2 x $CH_2$, 2'-CH, $(CD_3)_2SO$), 4.63 (3H, m, 5'-$CH_2$, 4'-CH), 5.65 (1H, m, 3'-CH), 6.37 (1H, m, 1'-CH), 6.72 (1H, d, J = 13.5Hz, CH=CHBr), 7.2-8.1 (10H, m, 2 x $C_6H_4$, 6-CH, CH=CHBr), 11.79 (1H, s, $D_2O$ exchangeable NH).

Analysis

Found: C, 60.49; H, 5.67; N, 4.27 %. $C_{33}H_{37}BrN_2O_7$ requires C, 60.65; H, 5.71; N, 4.29 %.

After a trace component the next product to elute was E-5-(2-bromovinyl)-5'-O-(4-n-butylbenzoyl)-2'-deoxyuridine (Example 40) (31%), recrystallised from ethyl acetate, m.p. 182-183°C; Rf 0.34 (chloroform/ethanol 20:1); νmax (KBr) 3500-2800 (NH, OH), 1720, 1710, 1655 (C=O) $cm^{-1}$; $^1$Hnmr [$(CD_3)_2SO$] δ 0.90 (3H, t, J = 6Hz, $CH_3$), 1.43 (4H, m, 2 x $CH_2$), 2.49 (4H, m, $CH_2$, 2'-$CH_2$, $(CD_3)_2SO$), 4.11 (1H, m, 4'-CH), 4.50 (3H, m, 5'-$CH_2$, 3'-CH), 5.50 (1H, br.s, $D_2O$ exchangeable 3'-OH), 6.26 (1H, m, 1'-CH), 6.78 (1H, d, J = 13.5Hz, CH=CHBr), 7.2-8.1 (6H, m, $C_6H_4$, 6-CH, CH=CHBr), 11.70 (1H, s, $D_2O$ exchangeable, NH).

Analysis

Found: C, 53.22; H, 5.34; N, 5.56 %. $C_{22}H_{25}BrN_2O_6$ requires C, 53.55; H, 5.06; N, 5.68 %.

BAD ORIGINAL

Examples 41, 42 and 43

E-5-(2-Bromovinyl)-3',5'-di-O-(4-N,N-dimethylaminobenzoyl)-2'-deoxyuridine, E-5-(2-Bromovinyl)-3'-O-(4-N,N-dimethylaminobenzoyl)-2'-deoxyuridine and E-5-(2-Bromovinyl)-5'-O-(4-N,N-dimethylaminobenzoyl)-2'-deoxyuridine

Reaction of E-5-(2-bromovinyl)-2'-deoxyuridine with p-N,N-dimethylaminobenzoyl chloride for 22 hours gave a mixture of products which were separated by column chromatography, eluting initially with chloroform and increasing polarity to chloroform/ethanol 20:1. The first component to elute was E-5-(2-bromovinyl)-3',5'-di-O-(4-N,N-dimethylaminobenzoyl)-2'-deoxyuridine (Example 41) (13.5%), recrystallised chloroform/acetone, m.p. 244-245$^{o}$C; Rf 0.23 in ethyl acetate/cyclohexane 1:1; νmax (KBr) 3300-2800 (NH), 1735, 1700, 1675 (C=O) cm$^{-1}$; $^{1}$Hnmr [$(CD_3)_2SO$] δ 2.50 (2H, m, 2'-$CH_2$, $(CD_3)_2SO$), 3.00 (12H, s, 2 x $(CH_3)_2N$), 4.50 (3H, m, 5'-$CH_2$, 4'-CH), 5.58 (1H, m, 3'-CH), 6.30 (1H, m, 1'-CH), 6.5-8.0 (11H, m, 2 x $C_6H_4$, 6-CH, CH=CHBr), 11.70 (1H, s, NH).

Analysis

Found: C, 55.20; H, 4.74; N, 8.77 %. $C_{29}H_{31}BrN_4O_7$ requires C, 55.51; H, 4.98; N, 8.93 %.

The second component to elute was E-5-(2-bromovinyl)-3'-O-(4-N,N-dimethylaminobenzoyl)-2'-deoxyuridine (Example 42) (6%), recrystallised from acetone, m.p. 227-228$^{o}$C; Rf 0.37 (ethyl acetate); νmax (KBr) 3500-2800 (NH, OH), 1710, 1680 (C=O) cm$^{-1}$; $^{1}$Hnmr [$(CD_3)_2SO$] δ 2.50 (2H, m, 2'-$CH_2$, $(CD_3)_2SO$), 3.05 (6H, s, $(CH_3)_2N$), 3.75 (2H, m, 5'-$CH_2$), 4.18 (1H, m, 4'-CH), 5.25 (1H, m, $D_2O$ exchangeable, 5'-OH), 5.43 (1H, m, 3'-CH), 6.27 (1H, m, 1'-CH), 6.60-8.3 (7H, m, $C_6H_4$, CH=CHBr), 11.60 (1H, s, $D_2O$ exchangeable, NH).

Analysis

Found: C, 49.87; H, 4.58; N, 8.46 %. $C_{20}H_{22}BrN_3O_6$ requires C, 50.01; H, 4.62; N, 8.75 %.

Cont'd./....

The final component to elute was E-5-(2-bromovinyl)-5'-O-(4-N,N-dimethylaminobenzoyl)-2'-deoxyuridine (Example 43) (25%), recrystallised from ethyl acetate, m.p. 205-206°C (dec); Rf 0.33 (chloroform/ethanol 20:1); νmax (KBr) 3500-2900 (NH, OH), 1710, 1680, 1670, 1655 (C=O) $cm^{-1}$; $^1$Hnmr [$(CD_3)_2SO$] δ 2.20 (2H, m, 2'-$CH_2$), 3.00 (6H, s, $(CH_3)_2N$), 4.05 (1H, m, 4'-CH), 4.38 (3H, m, 5'-$CH_2$, 3'-CH), 5.45 (1H, br.s, $D_2O$ exchangeable, 3'-OH), 6.20 (1H, m, 1'-CH), 6.5-8.0 (7H, m, $C_6H_4$, 6-CH, CH=CHBr), 11.63 (1H, s, $D_2O$ exchangeable, NH).

Analysis

Found: C, 50.34; H, 4.27; N, 8.52 %. $C_{20}H_{22}BrN_3O_6$ requires C, 50.01; H, 4.62; H, 8.75 %.

BAD ORIGINAL

Example 44

E-5-(2-Bromovinyl)-5'-O-(10-undecenoyl)-2'-deoxyuridine

10-Undecenoyl chloride (2.2mL) was added slowly over 0.5h to a stirred solution of E-5-(2-bromovinyl)-2'-deoxyuridine (3g) in pyridine (50mL) at $0^{o}$. The mixture was then allowed to come to room temperature and stirred at room temperature for 2 hours. The mixture was partitioned between aqueous hydrochloric acid (2M)-ethyl acetate. The organic extract was washed with aqueous sodium bicarbonate, dried and evaporated to give a solid which was then chromatographed over silica gel (160g). Elution of the column with ethyl acetate-hexane (3:1) gave E-5-(2-bromovinyl)-5'-O-(10-undecenoyl)-2'-deoxyuridine (2.5g, 56%), m.p. 150-151$^{c}$C (from ethyl acetate/acetone); λmax (EtOH) 250 (ε 14,500) and 293 (ε 12,400) nm; νmax (KBr) 1730, 1715, 1681, 1669, 1650, 1280, 1172 and 1165 $cm^{-1}$; $^{1}$Hnmr [$(CD_3)_2SO$] δ 1.05-1.75 (12H, m), 1.83-2.43 (6H, m), 3.94 (1H, m, 4'-CH), 4.25 (3H, m, 3'-CH, 5'-$CH_2$), 4.82-5.15 (2H, m), 5.40 (1H, m, $D_2O$ exchangeable, 3'-OH), 5.57-6.05 (1H, m), 6.17 (1H, t, J = 6Hz, 1'-CH), 6.93 (1H, d, J = 14Hz, C<u>H</u>=CHBr), 7.34 (1H, d, J = 14Hz, CH=C<u>H</u>Br), 7.80 (1H, s, 6-CH), 11.57 (1H, m, $D_2O$ exchangeable, NH).

Analysis

Found: C, 53.05; H, 6.5; N, 5.6 %. $C_{22}H_{31}N_2O_6Br$ requires C, 52.9; H, 6.25; N, 5.6 %.

Example 45

E-5-(2-Bromovinyl)-5'-O-(1-adamantanecarbonyl)-2'-deoxyuridine

1-Adamantanecarboxylic acid chloride (2.8g) was added to a stirred solution of E-5-(2-bromovinyl)-2'-deoxyuridine (3g) in pyridine (50mL) at $0^{O}$. The mixture was allowed to come to room temperature and stirred at room temperature for 4.5h. It was then partitioned between aqueous hydrochloric acid (5M)-ethyl acetate (1:1, 500mL). The organic extract was further washed with aqueous hydrochloric acid, aqueous sodium bicarbonate, brine, dried, and evaporated. The residue was chromatographed over silica gel (150g). Elution of the column with ethyl acetate-hexane (4:1) gave E-5-(2-bromovinyl)-5'-O-(1-adamantanecarbonyl)-2'-deoxyuridine (1.2g, 27%), m.p. 230-232$^{O}$C (from ethyl acetate-hexane); λmax (EtOH) 250 (ε 13,900) and 292 (ε 11,900) nm; νmax (KBr) 1725, 1698, 1290, 1075, and 535 $cm^{-1}$; $^{1}$Hnmr [$(CD_3)_2SO$] δ 1.53-2.05 (15H, m, Ad-H), 2.20 (2H, t, J = 6Hz, 2'-$CH_2$), 3.95 (1H, m, 4'-CH), 4.20 (3H, m, 3'-CH, 5'-$CH_2$), 5.40 (1H, m, $D_2O$ exchangeable, 3'-OH), 6.13 (1H, t, J = 6Hz, 1'-CH), 6.90 (1H, d, J = 14Hz, CH=CHBr), 7.30 (1H, d, J = 14Hz, CH=CHBr), 7.74 (1H, s, 6-CH), and 11.64 (1H, m, $D_2O$ exchangeable, 3-NH)

(Found: $M^+$ 494.1039. $C_{22}H_{27}BrN_2O_6$ requires $M^+$ 494.1029).

0082668

Example 46

E-5-(2-Bromovinyl)-3-N-(p-methoxybenzoyl)-3'-O-(p-methoxybenzoyl)-2'-deoxyuridine

To a solution of E-5-(2-bromovinyl)-5'-O-dimethoxytrityl-2'-deoxyuridine (0.58g, 0.9mmol) in dry dioxan (10mL) and triethylamine (2.5mL, 18mmol), p-methoxybenzoyl chloride (1mL, 7.3mmol) was added. The solution was stirred at room temperature overnight, then triethylamine (5mL) added and the mixture poured into ice/water (100mL) and extracted with three 50ml portions of ethyl acetate. The organic phase was washed with ammonium chloride solution, sodium carbonate solution and finally water and dried over magnesium sulphate. After removal of the solvent the product was columned on silica eluting with ethyl acetate/cyclohexane 1:2 to give E-5-(2-bromovinyl)-5'-O-dimethoxytrityl-3-N-(p-methoxybenzoyl)-3'-O-(p-methoxybenzoyl)-2'-deoxyuridine (0.64g, 79%);

$\nu_{max.}$ (KBr) 1745, 1705, 1660 (C=O) $cm^{-1}$;

$^1H$ nmr ($CDCl_3$) $\delta$ 2.65 (2H, m, 2'-$CH_2$), 3.58 (2H, m, 5'-$CH_2$), 3.83 (6H, s, $CH_3O$), 3.89 (6H, s, $CH_3O$), 4.35 (1H, m, 4'-CH), 5.80 (2H, m, 3'-CH and CH=CHBr), 6.50 (1H, m, 1'-CH), 6.6-8.1 (23H, m, 4x$C_6H_4$, $C_6H_5$, CH=CHBr and 6-CH).

A solution of E-5-(2-bromovinyl)-5'-O-dimethoxytrityl-3-N-(p-methoxybenzoyl)-3'-O-(p-methoxybenzoyl)-2'-deoxyuridine (0.92g, 1mmol) in 80% acetic acid (40mL) was stirred at room temperature for 45 mins. The solvent was removed and the residue columned on silica eluting with ethyl acetate/cyclohexane 1:3 to give E-5-(2-bromovinyl)-3-N-(p-methoxybenzoyl)-3'-O-(p-methoxybenzoyl)-2'-deoxyuridine Example 46 (0.4g,

64%), recrystallised from ethanol, mp 115-116°C; $\nu_{max.}$ (KBr) 3500 (OH), 1740, 1705, 1665, 1600 (C=O)$cm^{-1}$

$^1H$ nmr [$(CD_3)_2SO$] $\delta$ 2.48 (2H, m, 2'-$CH_2$), 3.87 (8H, m, 2x$CH_3O$, 5'-$CH_2$), 4.25 (1H, m, 4'-CH), 5.34 (1H, br.s, $D_2O$ exchangeable, 5'-OH), 5.50 (1H, m, 3'-CH), 6.28 (1H, m, 1'-CH), 6.8-8.5 (11H, m, 2x$C_6H_4$, 6-CH, CH=CHBr).

Analysis

Found: C, 53.71; H, 4.18; N, 4.55%. $C_{27}H_{25}BrN_2O_9$ requires: C, 53.92; H, 4.19; N, 4.66%.

Example 47

E-5-(2-Bromovinyl)-3-N-(p-chlorobenzoyl)-3'-O-(p-chlorobenzoyl)-2'-deoxyuridine

A solution of E-5-(2-bromovinyl)-5'-O-dimethoxytrityl-2'-deoxyuridine (0.6g, 0.95mmol) in dry dioxan (10mL) was treated with triethylamine (2.15mL, 15.5mmol) and p-chlorobenzoyl chloride (0.72mL, 5.7mmol) in portions over three days. Triethylamine (5mL) was added and the mixture was poured into ice/water (100mL) and extracted with three 50mL portions of ethyl acetate. The organic phase was washed with ammonium chloride solution, sodium carbonate solution and water and dried over magnesium sulphate. After removal of the solvent the residue was columned on silica eluting with ethyl acetate/cyclohexane 1:2 to give E-5-(2-bromovinyl)-3-N-(p-chlorobenzoyl)-3'-O-(p-chlorobenzoyl)-5'-O-dimethoxytrityl-2'-deoxyuridine (0.7g, 81%);

$\nu_{max.}$ (KBr) 1755, 1715, 1670 (C=O) $cm^{-1}$;

$^1H$ nmr [$(CD_3)_2SO$] $\delta$ 2.67 (2H, m, 2'-$CH_2$), 3.40 (2H, m, 5'-$CH_2$), 3.73 (6H, s, 2x$CH_3O$), 4.30 (1H, m, 4'-CH), 5.53 (1H, m, 3'-CH), 6.30 (1H, m, 1'-CH), 6.57 (1H, d, J=13.5Hz, C<u>H</u>=CHBr), 6.7-8.3 (23H, m, 4x$C_6H_4$, $C_6H_5$, 6-CH, CH=C<u>H</u>Br).

A solution of E-5-(2-bromovinyl)-3-N-(p-chlorobenzoyl)-3'-O-(p-chlorobenzoyl)-5'-O-dimethoxytrityl-2'-deoxyuridine (0.7g, 0.8mmol) in 80% acetic acid (40mL) was stirred at room temperature for 45 mins. The solvent was removed and the residue columned on silica eluting with ethyl acetate/cyclohexane 1:3 to give E-5-(2-bromovinyl)-3-N-(p-chlorobenzoyl)-3'-O-(p-chlorobenzoyl)-2'-deoxyuridine (Example 47) (0.28g,

68%), recrystallised from ethanol/water, mp 112-113°C; $\nu$max.(KBr) 3450 (OH), 1750, 1710, 1670 (C=O) $cm^{-1}$;

$^1H$ nmr [$(CD_3)_2SO$] $\delta$ 2.60 (2H, m, 2'-$CH_2$), 3.83 (2H, m, 5'-$CH_2$), 4.30 (1H, m, 4'-CH), 5.40 (3H, m, 3'-CH and $D_2O$ exchangeable, 5'-OH), 6.26 (1H, m, 1'-CH) and 6.8-9.1 (11H, m, 2x$C_6H_4$, 6-CH and CH=CHBr).

Analysis

Found: C, 49.07; H, 2.82; N, 4.37%. $C_{25}H_{19}BrCl_2N_2O_7$ requires: C, 49.21; H, 3.14; N, 4.59%.

Example 48

E-5-(2-Bromovinyl)-3-N-(p-dimethylaminobenzoyl)-3'-O-(p-dimethylaminobenzoyl)-2'-deoxyuridine

To a solutionof E-5-(2-bromovinyl)-5'-O-dimethoxytrityl-2'-deoxyuridine (1.03g, 1.6mmol) in dry dioxan (20mL) and triethylamine (4.5mL, 32mmol), p-N,N-dimethylaminobenzoyl chloride (2.4g, 13mmol) was added. The mixture was stirred at room temperature for two days. Triethylamine (5mL) was added and the mixture was poured into ice/water (100mL) and extracted with one 100mL and two 50mL portions of ethyl acetate. The organic phase was washed with ammonium chloride solution, sodium carbonate solution and water, and dried over magnesium sulphate. After removal of the solvent the residue was columned on silica eluting with ethyl acetate/cyclohexane 1:1 to give E-5-(2-bromovinyl)-5'-O-dimethoxytrityl-3-N-(p-dimethylaminobenzoyl)-3'-O-(p-dimethylaminobenzoyl)-2'-deoxyuridine (1.3g, 86%);

$\nu_{max.}$ (KBr) 1755, 1735, 1700, 1670 (C=O) $cm^{-1}$;

$^1H$ nmr [$(CD_3)_2SO$] $\delta$ 2.50 (2H, m, 2'-$CH_2$), 3.00 (12H, s, 4x$CH_3N$), 3.35 (2H, m, 5'-CH), 3.85 (6H, s, 2x$CH_3O$), 4.35 (1H, m, 4'-CH), 5.60 (1H, m, 3'-CH), 6.40 (1H, m, 1'-CH), 6.6-8.3 (24H, m, 4x$C_6H_4$, $C_6H_5$, 6-CH, CH=CHBr).

A solution of E-5-(2-bromovinyl)-5'-O-dimethoxytrityl-3-N-(p-dimethylaminobenzoyl)-3'-O-(p-dimethylaminobenzoyl)-2'-deoxyuridine (1.3g, 14mmol) in 80% acetic acid (80mL) was stirred at room temperature for 30 mins. The solvent was removed and the residue columned on silica. Elution with ethyl acetate/cyclohexane 1:3 gave after recovery of unreacted starting material (0.39g), E-5-(2-bromovinyl)-3-N-(p-dimethyl-

aminobenzoyl)-3'-O-(p-dimethylaminobenzoyl)-2'-deoxyuridine (Example 48) (0.26g, 43%); recrystallised from ethanol/water, mp 140-141°C;

$\nu_{max}$ (KBr) 3450 (OH), 1730, 1700, 1665, 1600 (C=O) $cm^{-1}$;

$^1H$ nmr [$(CD_3)_2SO$] $\delta$ 2.54 (2H, m, 2'-$CH_2$), 3.0 (6H, s, 2x$CH_3$), 3.1 (6H, s, 2x$CH_3$), 3.8 (2H, m, 5'-$CH_2$), 4.2 (1H, m, 4'-CH), 5.3 (2H, m, 3'-CH, $D_2O$ exchangeable, 5'-OH), 6.28 (1H, m, 1'-CH), 6.6-8.4 (11H, m, 2x$C_6H_4$, 6-CH, CH=CHBr).

Analysis

Found: C, 55.56; H, 4.83; N, 8.69%. $C_{29}H_{31}BrN_4O_6$ requires: C, 55.51; H, 4.98; N, 8.93%.

Example 49

E-5-(2-Bromovinyl)-3-N-(o-methylbenzoyl)-3'-O-(o-methyl benzoyl-2'-deoxyuridine

To a solution of E-5-(2-bromovinyl)-5'-O-dimethoxy trityl-2'-deoxyuridine (0.83g, 1.3mmol) in dioxan (10mL) and triethylamine (3.6mL, 26mmol) was added o-methylbenzoyl chloride (0.68mL, 5.2mmol). The mixture was stirred overnight at room temperature, and since acylation was incomplete additional o-methylbenzoyl chloride (0.68mL, 5.2mmol) was added, and stirring was continued for another 24 hours. Triethylamine (5mL) was added and the mixture was then poured into ice/water (100mL) and extracted with three 50mL portions of ethyl acetate. The organic phase was washed with ammonium chloride solution, sodium carbonate solution and water and dried with magnesium sulphate. The solvent was removed and the residue was columned on silica eluting with ethyl acetate/cyclohexane 1:6 to give E-5-(2-bromovinyl)-5'-O-dimethoxytrityl-3-N-(o-methylbenzoyl)-3'-O-(o-methylbenzoyl)-2'-deoxyuridine (0.8g, 72%);

$\nu_{max}$ (KBr) 1750, 1710, 1670 (C=O) $cm^{-1}$;

$^1H$ nmr [$(CD_3)_2SO$] $\delta$ 2.60 (8H, m, 2'-$CH_2$, 2x$CH_3$, $(CD_3)_2SO$), 3.37 (2H, m, 5'-$CH_2$), 3.70 (6H, s, 2x$CH_3O$), 4.27 (1H, m, 4'-CH), 5.50 (1H, m, 3'-CH), 6.24 (1H, m, 1'-CH), 6.50 (1H, d, J=13.5Hz, C<u>H</u>=CHBr), 6.7-8.1 (23H, m, 4x$C_6H_4$, $C_6H_5$, 6-CH, CH=C<u>H</u>Br).

A solution of E-5-(2-bromovinyl)-5'-O-dimethoxytrityl-3-N-(o-methylbenzoyl)-3'-O-(o-methylbenzoyl)-2'-deoxyuridine (0.9g, 1mmol) in 80% glacial acetic acid (40mL) was stirred at room temperature for 45 mins. The solvent was removed and the residue columned

on silica eluting with ethyl acetate/cyclohexane 1:4 to give E-5-(2-bromovinyl)-3-N-(o-methylbenzoyl)-3'-O-(o-methylbenzoyl)-2'-deoxyuridine (Example 49) (0.44g, 75%) as a foam;

$\nu$max (KBr) 3450 (OH), 1750, 1705, 1665, 1600 (C=O) $cm^{-1}$;

$^1H$ nmr $\delta$ [$(CD_3)_2SO$] 2.60 (8H, m, 2x$CH_3$, 2'-$CH_2$), 3.80 (2H, m, 5'-$CH_2$), 4.28 (1H, m, 4'-CH), 5.34 (1H, t, J=5Hz, $D_2O$ exchangeable 5'-OH), 5.53 (1H, m, 3'-CH), 6.25 (1H, m, 1'-CH), 6.8-8.5 (11H, m, 2x$C_6H_4$, 6-CH, CH=CHBr).

Analysis

Found: C, 56.95; H, 4.46; N, 4.62%. $C_{27}H_{25}BrN_2O_7$ requires: C, 56.95; H, 4.43; N, 4.92%.

## Antiviral Activity

### In Vitro

### Method

Vero (African Green Monkey Kidney) cells were grown to confluence in 6 well multidishes, each well being 3.5 cm in diameter. The cells were incubated with herpes simplex type 1 virus (HFEM strain) and overlaid with 0.5 mL of 0.9% agarose (w/v) in maintenance medium containing the test compound at a range of concentrations from 100 μg/mL in half-log dilution steps. The virus infected cultures were then incubated at 37°C for 6 days before fixing in 4% formaldehyde solution and staining with carbolfuchsin. The dishes were then examined to find the concentration of test compound causing a 50% reduction in the number of virus plaques formed ($PDD_{50}$ value) and the minimum concentration of test compound which killed the cell monolayer, leaving a clear zone devoid of cells and virus plaques (MTD).

Results

| Example No. | $PDD_{50}$ | | MTD (µg/mL) |
|---|---|---|---|
| | µg/µL | µM | |
| 2 | 54.0 | 100 | >100 |
| 3 | 1.7 | 3.7 | >100 |
| 10 | 2.1 | 3.8 | 100 |
| 11 | 1.6 | 3.7 | >100 |
| 12 | 0.23 | 0.53 | 100 |
| 15 | 74 | 121 | >100 |
| 17 | 3.9 | 6.9 | >100 |
| 18 | 0.84 | 1.78 | >100 |
| 19 | 6.3 | 13.5 | 100 |
| 20 | 0.12 | 0.25 | >100 |
| 21 | 0.25 | 0.54 | >100 |
| 22 | 0.24 | 0.50 | >100 |
| 23 | 0.26 | 0.62 | >100 |
| 24 | 0.46 | 1.10 | >100 |
| 25 | 0.32 | 0.72 | >100 |
| 26 | 0.25 | 0.53 | >100 |
| 29 | 1.12 | 1.87 | >100 |
| 30 | 0.11 | 0.18 | >100 |
| 31 | 20 | 32 | >100 |
| 32 | 0.95 | 2.0 | >100 |
| 33 | 100 | 147 | >100 |
| 34 | 1.2 | 2.3 | >100 |
| 35 | 1.4 | 2.8 | >100 |
| 37 | 0.63 | 1.4 | >100 |
| 38 | 0.78 | 1.7 | >100 |
| 40 | 6.1 | 12 | >100 |
| 42 | 0.63 | 1.4 | >100 |
| 48 | 0.47 | 0.94 | >100 |
| 49 | 0.55 | 1.1 | 100 |

CLAIMS

1. A compound of formula (I):

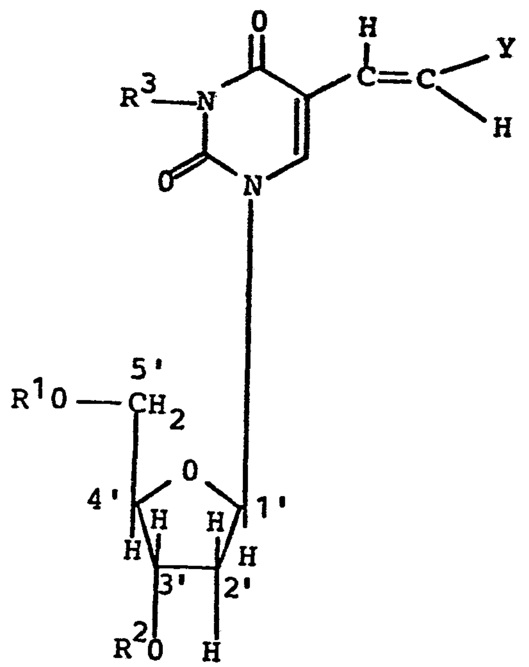

(I)

wherein Y is a hydrogen, chlorine, bromine or iodine atom, each of $R^1$, $R^2$ and $R^3$ is a hydrogen atom or an acyl radical of the formula

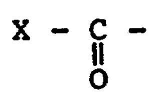

in which X is optionally unsaturated straight or branched chain $C_{1-18}$ alkyl, mono- or polycyclo $C_{1-18}$ alkyl, $C_{1-18}$ cycloalkylalkyl, phenyl optionally substituted with $C_{1-6}$ alkyl, $C_{1-6}$ alkoxy, nitro, optionally $C_{1-6}$ alkyl substituted amino, halogen, or N,N-dialkylsulphonamido, or is straight or branched chain phenyl alkyl optionally substituted in the phenyl ring with $C_{1-6}$ alkyl, $C_{1-6}$ alkoxy, or halogen, with the provisos that at

least one of $R^1$, $R^2$ and $R^3$ is an acyl radical, and that when $R^3$ is hydrogen, $R^1$ or $R^2$ is not $X-\underset{\underset{O}{\|}}{C}-$,

wherein X is $C_{1-6}$ alkyl.

2. A compound as claimed in claim 1, characterised in that Y is a bromine atom.

3. A compound as claimed in claims 1 or 2, characterised in that X is a phenyl radical optionally substituted in the para position.

4. A compound as claimed in any one of claims 1 to 3, characterised in that X is a straight, or branched chain $C_{1-6}$ alkyl group, or is a cycloalkyl or cycloalkylalkyl group of up to twelve carbon atoms.

5. A compound as claimed in claim 1, characterised in that X is a $C_{7-18}$ straight chain alkyl group or a mono- or polycyclic $C_{7-18}$ alkyl group.

6. A process for preparing a compound of formula (I), as claimed in any one of claims 1 to 5, which comprises either the reaction or step wise reaction, of an optionally O-protected compound of formula (II):

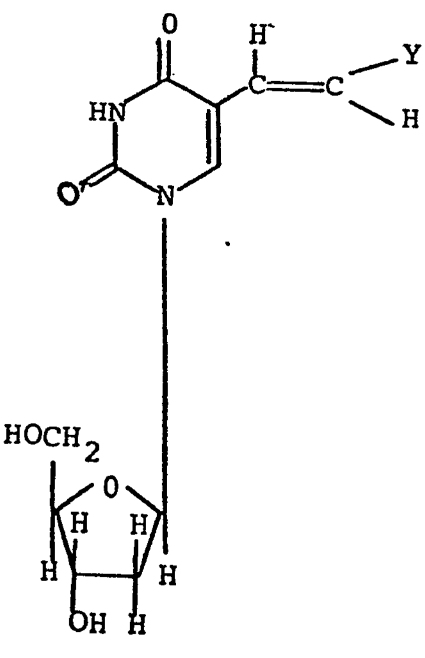

(II)

wherein Y is as defined in relation to formula (I), claim 1, with an acylating agent of formula X CO.L, wherein X is as defined in relation to formula (I), claim 1, and L is a conventional leaving group, and where necessary removing remaining protecting group(s), the O-protecting groups being conventional O-protecting groups, or which comprises selective hydrolysis of a di- or triacylated compound of formula (I).

7. A process for preparing a compound of formula (I) as claimed in claim 6, characterised in that the acylating agent is an acid halide or an anhydride.

8. A process for preparing a compound of formula (I) as claimed in claims 6 or 7, characterised in that the O-protecting group is a trityl group, a 4,4'-dimethoxytrityl group, or is a tri-lower alkyl silyl group.

9. A pharmaceutical composition comprising a compound of formula (I), as claimed in any one of claims 1 to 5, together with a pharmaceutically acceptable excipient or carrier.

0082668

Application number

European Patent Office

# EUROPEAN SEARCH REPORT

EP 82 30 6686

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | GB-A-2 060 604 (THE UNIVERSITY OF BIRMINGHAM) *Page 1* | 1,5 | C 07 H 19/08<br>A 61 K 31/70 |
| Y | DE-A-3 010 399 (GAURI, K.) *Pages 1,2* | 1,5 | |
| Y | EP-A-0 009 882 (FUNAI) *Pages 1-4* | 1,5 | |
| Y | GB-A-2 072 164 (FUNAI) *Pages 1-3* | 1,5 | |
| Y,P | EP-A-0 061 283 (BEECHAM) *Pages 1-4* | 1,5 | |
| | ----- | | TECHNICAL FIELDS SEARCHED (Int. Cl. ³) |
| | | | C 07 H 19/00<br>A 61 K 31/00 |

The present search report has been drawn up for all claims

| Place of search | Date of completion of the search | Examiner |
|---|---|---|
| THE HAGUE | 21-02-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82